# EUROPEAN PATENT SPECIFICATION

(11) **EP 0 675 924 B1**
(45) Date of publication and mention of the grant of the patent: **12.12.2001**
(21) Application number: 94914173.3
(22) Date of filing: 13.04.1994
(51) Int. Cl.: C09B 23/02, C07D 215/18, C07D 213/61, C07D 213/64, C07D 215/22, G01N 33/52, G01N 33/58, G01N 31/22, G01N 21/64, G01N 27/26, C12Q 1/68

(54) **CYCLIC-SUBSTITUTED UNSYMMETRICAL CYANINE DYES**
RING-SUBSTITUIERTE, UNSYMMETRISCHE CYANINFARBSTOFFE
COLORANTS A BASE DE CYANINE ASSYMETRIQUE A SUBSTITUTION CYCLIQUE

(30) Priority: 13.04.1993 US 47683; 12.07.1993 US 90890; 01.11.1993 US 146328; 08.11.1993 US 148847
(43) Date of publication of application: 11.10.1995
(73) Proprietor: MOLECULAR PROBES INC, Eugene, OR 97402-9144 (US)
(72) Inventor: ROTH, Bruce, L., Corvallis, OR 97333 (US); MILLARD, Paul, J., Eugene, OR 97405 (US); YUE, Stephen, T., Eugene, OR 97402 (US); WELLS, K., Sam, Veneta, OR 97487 (US); HAUGLAND, Richard, P., Eugene, OR 97405 (US)
(74) Representative: Couchman, Jonathan Hugh
(86) International application number: PCT/US94/04127
(87) International publication number: WO 94/24213

(56) References cited:
- EP-A- 0 410 806
- EP-A- 0 453 197
- WO-A-93/06482
- GB-A- 2 074 340
- CHEMICAL ABSTRACTS, vol. 93, no. 26, 29 December 1980, Columbus, Ohio, US; abstract no. 241180j, M.A.KUDINOVA ET AL. 'Pyrylocyanines. 12. Unsymmetrical pyrylo-2-cyanines' page 91 ; & KHIM. GETEROTSIKL. SOEDIN. no. 7 , 1980 pages 903 - 908
- CHEMICAL ABSTRACTS, vol. 89, no. 14, 2 October 1978, Columbus, Ohio, US; abstract no. 112299y, J.SIMBERA ET AL 'Synthesis of polymethine dyes from 1-(3-chloro-2-tetrahydrofuryl)-4 -methylquinolinium chloride' page 151 ; & SER. FAC. SCI. NAT. UNIV PURKYNIANAE BRUN. vol. 8, no. 3 , 1978 pages 77 - 87

## Description

### FIELD OF THE INVENTION

The invention relates to fluorescent dyes for nucleic acids. In particular, the invention relates to dyes derived from unsymmetrical cyanine dyes having a saturated or unsaturated cyclic substituent that stain nucleic acids in a variety of media.

### BACKGROUND INFORMATION

In many fields of life sciences research, including biological, biomedical, genetic, fermentation, aquaculture, agricultural, forensic and environmental research, there is a need to identify nucleic acids both isolated and within cells as a routine component of standard experimental methods. Such applications require a fast, sensitive, and selective methodology that can detect nucleic acids, even when bounded (or surrounded) by cellular membranes, such as living cells. Additionally, analysis of cells from mixed populations of cells or microorganisms for both viability and/or Gram sign is a routine component of standard experimental methods.

Although certain unsymmetrical cyanine dyes were first described before the genetic role of nucleic acids was established (Brooker, et al., J. AM. CHEM. SOC. 64, 199 (1942)), a variety of unsymmetrical cyanine dyes have now been found to be very effective in the fluorescent staining of DNA and RNA. The compound sold as Thiazole Orange has particular advantages in the quantitative analysis of immature blood cells or reticulocytes US-A-4,883,867 to Lee, et al. (1989)) or in preferentially staining the nucleic acids of bloodborne parasites with little staining of nucleated blood cells US-A-4,937,198 to Lee, et al. (1990). Thiazole Orange and similar thioflavin dyes are permeant to many mammalian cells, yet are impermeant to some eukaryotic cells. WO-A-93,06482 discloses dimers of unsymmetrical fluorescent dyes useful for nucleic acid staining.

The inventors have discovered that attachment of various cyclic structures to a parent unsymmetrical cyanine produces a family of superior nucleic acid dyes. Surprisingly, although bulkier, the new dyes more quickly penetrate the cell membranes of a wider variety of cell types, including both gram-positive and gram-negative bacteria, yeasts, and eukaryotic cells as well as prokaryotic cells. The subject dyes also more rapidly stain electrophoretic gels used for the separation of nucleic acids. Direct comparison of the rate of uptake in bacteria with known dyes such as Thiazole Orange and its homologs, shows enhanced uptake of the new compounds (Table 1). Even in applications where cell permeability is not a factor, the quantum yield of most of these dyes is unexpectedly, and significantly, better than that of Thiazole Orange (Table 2).

Furthermore, by simple synthetic modification, a family of dyes having absorption and emission spectral properties that cover most of the visible and near-infrared spectrum can be prepared. The improved fluorescent properties of the dyes of the present invention present significant advantages for the detection of cellular or non-cellular nucleic acids in all areas of nucleic acid research. These dyes are particularly useful in combination with other dyes, for example to differentiate cells and/or determine viability.

**Table 1:**

| Loading Time | | | | |
|---|---|---|---|---|
| | To peak (sec) | | To Equilibrium (sec) | |
| DYE | *S. aureus* T_{0.5} | *E*. *coli* T_{0.5} | *S*. *aureus* T_{0.95} | *E*. *coli* T_{0.95} |
| **61** | 3.4 | 18.2 | 66.9 | 270.9 |
| **63** | 7.9 | ND | 172.2 | ND |
| **613** | 9.1 | 11.3 | 149.0 | 163.1 |
| **619** | 7.3 | 15.5 | 34.3 | 243.3 |
| **624** | 7.6 | 24.3 | 27.6 | 89.4 |
| **628** | 19.6 | 36.8 | 47.2 | 89.9 |
| **591** | 6.3 | 25.3 | 116.3 | 73.3 |
| **634** | 14.5 | 12.5 | 86.3 | 154.2 |
| **73** | 10.0 | 23.3 | 145.1 | 58.6 |
| **720** | 6.8 | 21.6 | 216.4 | 221.6 |
| **Thiazole Orange** | 57.2 | 39.2 | 242.0 | 125.9 |
| Loading time is expressed as: time required to reach half of the maximal fluorescence (T₀₅) and to reach 95% of the fluorescence measured at equilibrium (T₀₉₅). | | | | |

**Table 2:**

| Properties of Representative Dyes | | | | | | | |
|---|---|---|---|---|---|---|---|
| DYE | Ex/Em (nm) | | Properties on DNA | | | | RNA |
| | DNA¹ | RNA¹ | Kₚ² | QY³ | P.B.⁴ | F.E.⁵ | F.E.⁵ |
| **61** | 500/527 | 510/530 | 1.0E07 | 0.46 | 1.10 | 353 | 502 |
| **63** | 514/531 | 515/537 | 3.9E06 | 0.24 | 1.08 | 582 | 696 |
| **613** | 506/523 | 508/529 | 5.3E06 | 0.33 | 1.14 | 225 | 1614 |
| **619** | 488/317 | 492/529 | 9.7E06 | 0.62 | 0.89 | 301 | 518 |
| **624** | 480/501 | 485/505 | 5.0E06 | 0.58 | 1.17 | 661 | 1435 |
| **628** | 488/506 | 490/510 | 7.0E06 | 0.40 | 1.13 | 771 | 166 |
| **591** | 509/532 | 517/536 | 4.8E06 | 0.09 | 1.11 | 169 | 653 |
| **634** | 510/530 | 511/533 | 2.0E06 | 0.18 | 1.10 | 176 | 122 |
| **73** | 508/525 | 510/531 | 4.4E06 | 0.31 | 1.12 | 700 | 371 |
| **720** | 487/507 | 490/523 | 1.2E07 | 0.52 | 1.09 | 1330 | 107 |
| **Thiazole Orange** | 510/530 | 509/535 | 4.8E06 | 0.18 | 1.01 | 143 | 811 |

| | | | | | | | |
|---|---|---|---|---|---|---|---|
| 1. Obtained using a standard ratio of 50 µM bp of DNA (bases of RNA) to 1 µM dye (standard solution) in Tris buffered saline (10 mM Tris base, 1 mM EDTA and 50 mM NaCl), pH 7.4, in a spectrophotometer (absorbance), or in a fluorometer (emission) using 10-fold less dye and nucleic acid. | | | | | | | |
| 2. Partition coefficient (Kₚ) determined by linear fitting of plots of reciprocal fluorescence enhancement versus reciprocal DNA concentration, as measured using a CytoFluor microtiter plate fluorescence reader. | | | | | | | |
| 3. Quantum yield (QY) of dye on DNA (standard solution in Tris buffered saline adjusted to pH 10) in comparison with fluorescein (fluorescein assumed to have quantum yield of 0.92 under test conditions). | | | | | | | |
| 4. Photobleaching (P.B.), expressed as the residual fluorescence from the new dye relative to that of fluorescein under identical conditions. A 0.05 OD standard solution in Tris buffered saline is illuminated at 485 nm (ex. bandwidth of 20 nm), fluorescence is measured at time 0 and 30 min. Fraction of new dye fluorescence after 30 minutes is divided by fraction of fluorescein fluorescence under identical conditions. | | | | | | | |
| 5. Fluorescence enhancement (F.E.) is the fluorescence of the standard solution divided by the fluorescence of the same dye in the absence of nucleic acids. | | | | | | | |

### DESCRIPTION OF DRAWINGS

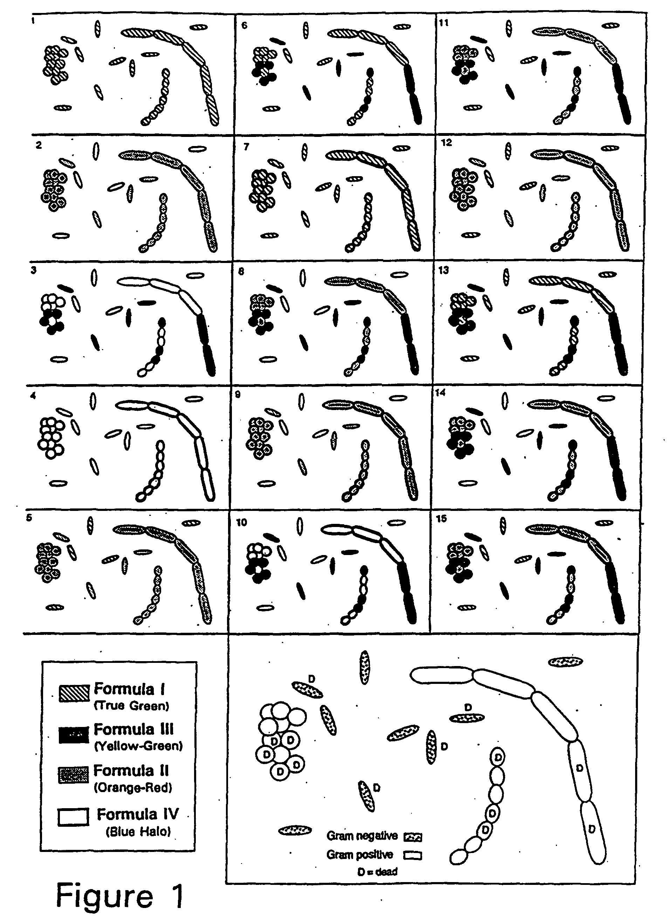

Figure 1: Each numbered panel (1-15) corresponds directly to the combination of stains shown in Table 5.

### SUMMARY OF THE INVENTION AND DESCRIPTION OF PREFERRED EMBODIMENTS

The cyclic-substituted unsymmetrical cyanine dyes of the invention are virtually non-fluorescent when diluted in aqueous solution. When bound to nucleic acid polymers such as DNA and RNA, however, the resultant dye-nucleic acid complex becomes extremely fluorescent upon illumination. The dyes of the present invention are highly permeant and label nucleic acids in a wide variety of solid or liquid samples, particularly in cells and gels. These dyes are optionally used in combination with other detection reagents to differentiate varioius properties of cells such as viability, Gram sign, or antibody staining.

The dyes of the invention comprise three parts: 1) a first heterocyclic ring system that is a substituted benzazolium ring system, 2) a linking methine bridge and 3) a second heterocyclic ring system that is a pyridinium or quinolinium ring system, one or more positions of which is substituted by a saturated or unsaturated, substituted or unsubstituted, cyclic substituent. The two ring systems are optionally further substituted independently by lower alkyl, ether, thioether, substituted or unsubstituted amine, sulfonate ester, halo, or cyclic substitutents. Preferably the ring nitrogen of the second heterocyclic ring system contains a cyclic subsitutent, adjacent to which is a second non-hydrogen substituent. The non-hydrogen substituent is preferably another cyclic substitutent, or a halo, an ether, a thioether, a substituted or unsubstituted amine, or a sulfonate ester substituent.

Specific examples of the dyes of the present invention are described by the formula: 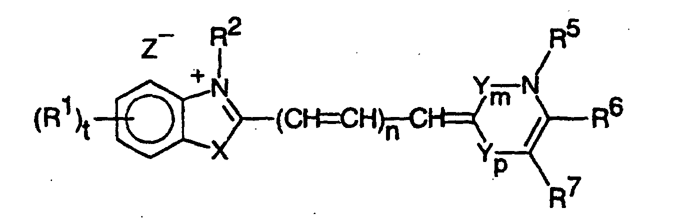 where the substituted benzazolium ring system on the left is linked by a methine bride to the righthand pyridinium or quinolinium ring system, one or more substituents of which must be an OMEGA.

An OMEGA is a saturated or unsaturated, substituted or unsubstituted, cyclic substituent that has a total of 2-16 ring carbon atoms in 1-2 alicyclic. aromatic, or heteroalicyclic or heteroaromatic rings containing 1-4 heteroatoms (wherein the hetero atoms are O, N or S) that is directly bonded to the pyridinium or quinolinium ring system by a single bond. Examples of OMEGA are substituted or unsubstituted cyclohexyls cyclohexenyls, morpholinos, and piperidinyls. Examples of OMEGA that are aromatic include substituted or unsubstituted naphthyls, phenyls, thienyls, benzothiazolyls, furanyls, oxazolyls, benzoxazolyls, and pyridinyls Substituents on OMEGA are independently hydrogen, halogen, alkyl, perfluoroalkyl, amino, alkylamino, dialkylamino, alkoxy or carboxyalkyl, each alkyl having 1-6 carbons. Preferred embodiments of OMEGA are substituted or unsubstituted naphthyl, phenyl, thienyl, morpholino, and cyclohexyl, more preferably substituted or unsubstituted phenyl.

Although R¹ on the benzazolium ring system is usually H, incorporation of one or more non-hydrogen substituents R¹ can be used to fine tune the absorption and emission spectrum of the resulting dye. For instance when R¹ is a methoxy (compound 770) its absorption spectrum shifts ∼ 12 nm and its emission spectrum shifts ∼ 18 nm (Table 5) relative to the comparable compound where R¹ is H (compound 63). The benzazole may contain more than one substituent R¹, which may be the same or different (t = 1-4). Each R¹ is optionally an alkyl group having from 1-6 carbons; or a trifluoromethyl; or a halogen; or -OR⁸, -SR⁸ or -(NR⁸R⁹) where R⁸ and R⁹, which can be the same or different, are independently H or alkyl groups having 1-6 carbons; or 1-2 alicyclic, aromatic, or heteroalicyclic or heteroaromatic rings having a total of 3-16 ring atoms (wherein the hetero atoms are O, N or S); or R⁸ and R⁹ taken in combination are -(CH₂)₂-L-(CH₂)₂- where L = -O-, -NR¹⁰, -CH₂- or a single bond where R¹⁰ is H or an alkyl group having 1-6 carbons. Typically, the compound contains no more than one R¹ that is not H.

The substituent R² is an alkyl group having 1-6 carbons, preferably methyl or ethyl, more preferably methyl.

The counterion Z⁻ is a biologically compatible ion that is stable and synthetically accessible. Examples of Z⁻ include, among others, chloride, bromide, iodide, sulfate, alkanesulfonate, arylsulfonate, phosphate, perchlorate, tetrafluoroborate, tetraarylboride, nitrate and anions of aromatic or aliphatic carboxylic acids. Preferred Z⁻ counterions are chloride, iodide, perchlorate and various sulfonates.

X is one of O, S, Se or NR¹⁵, where R¹⁵ is H or an alkyl group having 1-6 carbons. Alternatively, X is CR¹⁶R¹⁷, where R¹⁶ and R¹⁷, which may be the same or different, are independently H or alkyl groups having 1-6 carbons, or the carbons of R¹⁶ and R¹⁷ taken in combination complete a five or six membered saturated ring. Generally, R¹⁶ and R¹⁷ are methyls.

The two heterocyclic ring systems are linked by 1, 3 or 5 methine (-CH=) groups in such a way as to permit extensive electronic delocalization. When n = 0 the dyes are unsymmetrical monomethine dyes; when n = 1 the dyes are trimethine dyes; when n = 2, the dyes are pentamethine dyes. As with similar compounds (Griffiths, COLOUR AND CONSTITUTION OF ORGANIC MOLECULES, pp. 241 (1976)), the number of methine groups between the heteroaromatic rings influences the spectral properties of the dye (Table 3).

The N-bound substituent R⁵ is an alkyl, alkenyl, polyalkenyl, alkynyl or polyalkynyl group having 1-6 carbons; or R⁵ is an OMEGA. Most commonly R⁵ is an OMEGA.

The second ring system contains a ring fragment Y that is -CR³=CR⁴-, with subscripts p and m equal to 0 or 1, such that p + m = 1. For all embodiments, the ring contains a 6 membered pyridinium-based heterocycle according to one of these formulations 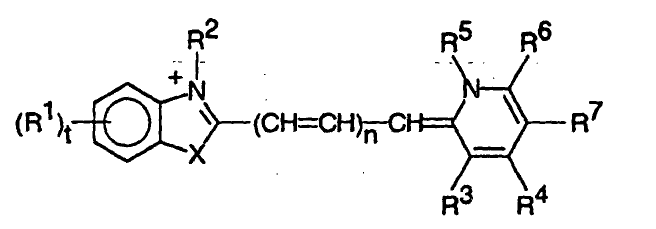 or 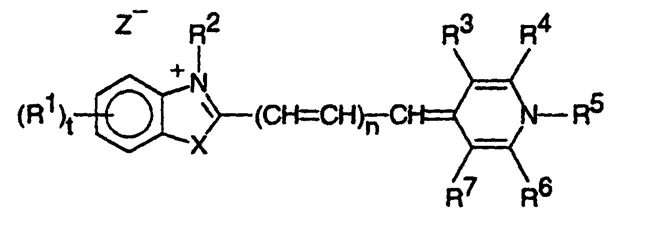 In preferred embodiments of the invention, m = 1 and p = 0 (4-pyridinium).

The substituents on the second heterocyclic ring system, R³, R⁴, R⁶ and R⁷, may be the same or different and are independently H; or an alkyl, alkenyl, polyalkenyl, alkynyl or polyalkynyl group having 1-6 carbons; or a halogen; or -OH, -OR⁸, -SR⁸, -(NR⁸R⁹), as defined previously; or -OSO₂R¹⁹ where R¹⁹ is alkyl having 1-6 carbons, or perfluoroalkyl having 1-6 carbons, or aryl; or an OMEGA (defined above); or R⁶ and R⁷ taken in combination are -(CH₂)ᵥ- where v = 3 or 4, forming a fused 5 or 6 membered ring, or R⁶ and R⁷, taken in combination form a fused 6 membered aromatic ring.

Where R⁶ and R⁷ taken in combination form a fused 6 membered aromatic ring, embodiments of this invention are quinolinium derivatives according to the formula 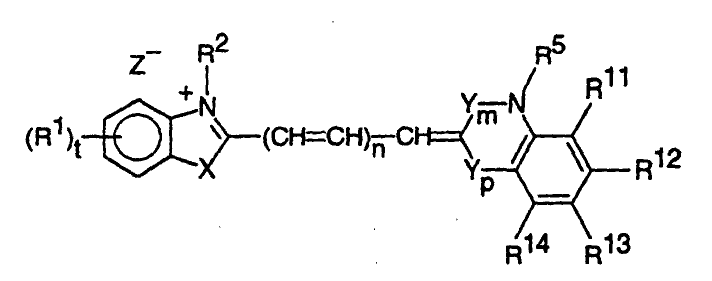 where ring substituents R¹¹, R¹², R¹³, and R¹⁴ may be the same or different, and are independently H; or an alkyl, alkenyl, polyalkenyl, alkynyl or polyalkynyl group having 1-6 carbons; or a halogen; or -OH, -OR⁸, -SR⁸, -(NR⁸R⁹), where R⁸ and R⁹ are as defined previously; or -OSO₂R¹⁹ where R¹⁹ is alkyl having 1-6 carbons, or perfluoroalkyl having 1-6 carbons, or aryl; or an OMEGA. A preferred embodiment of the invention is a quinolinium wherein m = 1 and p = 0 (4-quinolinium).

For all embodiments of the invention, one or more of the substituents of the pyridinium or quinolinium ring system is an OMEGA. Preferably, one or two substituents are OMEGAs. When more than one OMEGA is bound to a compound of the present invention, the two or more OMEGAs may be the same or different. For embodiments of the invention that contain pyridinium ring systems, OMEGA is preferably R⁵, or R⁶ or both. For embodiments of the invention that contain a 4-quinolinium ring system, OMEGA is preferably R⁴ or R⁵, or both. For embodiments of the invention that contain a 2-quinolinium ring system, OMEGA is preferably R⁵, R¹¹ or both. For all embodiments of the invention, preferably R⁵ is an OMEGA.

One embodiment of the invention contains exactly two non-hydrogen substituents on the second heterocyclic ring, one of which is an OMEGA. In one preferred embodiment, R⁵ is an OMEGA and the substituent adjacent to R⁵ (R⁶ for pyridiniums, R⁴ for 4-quinoliniums, and R¹¹ for 2-quinoliniums) is a non-hydrogen substituent. In one aspect, the substituent adjacent to R⁵ is halogen or -OSO₂R¹⁹, more preferably halogen. In another aspect, the substituent adjacent to R⁵ is an OMEGA. In another preferred embodiment, one non-hydrogen substituent is -OR⁸, -SR⁸, or -NR⁸R⁹, preferably -NR⁸R⁹.

**Table 3**

| DYE | $\frac{\text{EXmax}}{\text{EM max}}$ | QY (DNA) | QY (RNA) | Kp |
|---|---|---|---|---|
| Thiazole Orange | 510/530 | 0.18 | 0.15 | 4.8 E6 |
| 61 | 500/527 | 0.46 | 0.34 | 1.0 E7 |
| 63 | 514/531 | 0.24 | | 3.9 E6 |
| 64 | 450/523 | | | |
| 71 | 508/526 | 0.31 | | |
| 72 | 515/535 | 0.026 | | 1.2 E6 |
| 73 | 508/525 | 0.31 | | 4.4 E6 |
| 200 | 739/759 | | | |
| 542 | 510/527 | | | |
| 578 | 470/504 | | | 4.1 E5 |
| 582 | 516/533 | | | |
| 591 | 509/532 | 0.09 | 0.13 | 4.8 E6 |
| 613 | 506/523 | 0.33 | | 5.3 E6 |
| 616 | 471/510 | | | 3.8 E5 |
| 619 | 488/517 | 0.62 | 0.22 | 9.7 E6 |
| 621 | 635/656 | | | |
| 624 | 480/501 | 0.58 | 0.57 | 5.0 E6 |
| 628 | 488/506 | 0.40 | | 7.0 E6 |
| 630 | 517/544 | 0.19 | | |
| 633 | 489/508 | 0.12 | | 7.4 E5 |
| 634 | 510/530 | 0.18 | | 2.0 E6 |
| 637 | 601/622 | 0.28 | | |
| 639 | 513/548 | 0.20 | | 8.0 E6 |
| 640 | 471/516 | | | |
| 641 | 503/526 | 0.35 | | 2.0 E7 |
| 672 | 586/611 | | | |
| 720 | 487/507 | 0.52 | | 1.2 E7 |
| 742 | 570/611 | | | |
| 752 | 494/518 | 0.51 | | |
| 758 | 504/524 | 0.44 | | 8.5 E6 |
| 760 | 483/510 | 0.68 | | |
| 764 | 486/508 | 0.58 | 0.46 | 1.1 E7 |
| 765 | 506/524 | 0.50 | | 1.1 E7 |
| 770 | 526/549 | | | 1.7 E6 |
| 774 | 517/533 | | | 7.9 E6 |
| 776 | | 0.65 | | |
| 780 (Cl) | 513/536 | 0.09 | | 3.4 E6 |
| 780 (S) | | 0.31 | | |
| 830 | 517/533 | | | |
| 834 | 486/507 | | | |
| 835 | 495/518 | | | |
| 853 | 516/555 | | | |
| 854 | 483/520 | | | |
| 856 | 502/523 | 0.43 | | |
| 5103 | 511/530 | 0.18 | | 5.4 E6 |
| 6104 | 505/523 | 0.52 | | 1.3 E7 |

**Table 4**

| DYE# | X | heterocycle | R¹ | R² | R⁴ | R⁵ | R¹¹ | R¹² | n |
|---|---|---|---|---|---|---|---|---|---|
| 125 | S | 2-pyridinium | H | Me | H | phenyl | - | - | 0 |
| 578 | S | 4-pyridinium | H | Me | Cl | phenyl | - | - | 0 |
| 616 | S | 4-pyridinium | H | Me | Cl | *o*-MeO-phenyl | - | - | 0 |
| 640 | S | 4-pyridinium | H | Me | H | phenyl | - | - | 0 |
| 742 | S | 4-pyridinium | H | Me | n-butyl | phenyl | - | - | 1 |
| 64 | S | 2-quinolinium | H | Me | H | phenyl | H | H | 0 |
| 61 | S | 4-quinolinium | H | Me | n-butyl | phenyl | H | H | 0 |
| 63 | S | 4-quinolinium | H | Me | H | phenyl | H | H | 0 |
| 71 | S | 4-quinolinium | H | Me | n-butyl | thienyl | H | H | 0 |
| 72 | S | 4-quinolinium | H | Me | H | Me | phenyl | H | 0 |
| 73 | S | 4-quinolinium | H | Me | H | cyclohexyl | H | H | 0 |
| 130 | S | 4-quinolinium | H | Me | -NH-phenyl | phenyl | H | H | 0 |
| 100 | S | 4-quinolinium | H | Me | n-butyl | phenyl | H | H | 2 |
| 200 | S | 4-quinolinium | H | Et | Cl | phenyl | H | H | 0 |
| 542 | S | 4-quinolinium | H | Me | H | cyclohexenyl | H | H | 0 |
| 582 | S | 4-quinolinium | H | Me | Cl | *p*-MeO-phenyl | H | H | 0 |
| 591 | S | 4-quinolinium | H | Me | Cl | phenyl | H | H | 0 |
| 613 | S | 4-quinolinium | H | Me | Me | phenyl | H | H | 0 |
| 619 | S | 4-quinolinium | H | Me | -NEt₂ | phenyl | H | H | 0 |
| 621 | S | 4-quinolinium | H | Me | n-butyl | phenyl | H | H | 1 |
| 624 | O | 4-quinolinium | H | Me | n-butyl | phenyl | H | H | 0 |
| 628 | S | 4-quinolinium | H | Me | -OMe | phenyl | H | H | 0 |
| 630 | S | 4-quinolinium | H | Me | phenyl | phenyl | H | H | 0 |
| 633 | O | 4-quinolinium | H | Me | Cl | phenyl | H | H | 0 |
| 634 | S | 4-quinolinium | H | Me | H | n-hexyl | H | H | 0 |
| 637 | O | 4-quinolinium | H | Me | n-butyl | phenyl | H | H | 1 |
| 639 | S | 4-quinolinium | H | Me | phenyl | Me | H | H | 0 |
| 641 | S | 4-quinolinium | H | Me | -SMe | phenyl | H | H | 0 |
| 672 | O | 4-quinolinium | H | Me | -OMe | phenyl | H | H | 1 |
| 720 | S | 4-quinolinium | H | Me | -OEt | phenyl | H | H | 0 |
| 752 | S | 4-quinolinium | H | Me | morpholinyl | Me | H | H | 0 |
| 758 | S | 4-quinolinium | Cl | Me | n-butyl | phenyl | H | H | 0 |
| 760 | S | 4-quinolinium | H | Me | -NEt₂ | phenyl | H | -OMe | 0 |
| 764 | S | 4-quinolinium | H | Me | -O-iPr | phenyl | H | H | 0 |
| 765 | S | 4-quinolinium | H | Me | cyclohexyl | phenyl | H | H | 0 |
| 770 | S | 4-quinolinium | -OMe | Me | H | phenyl | H | H | 0 |
| 774 | S | 4-quinolinium | H | Me | Br | phenyl | H | H | 0 |
| 776 | S | 4-quinolinium | H | Me | -N-nPr₂ | phenyl | H | H | 0 |
| 780 (Cl) | S | 4-quinolinium | H | Me | Cl | cyclohexyl | H | H | 0 |
| 780 (S) | S | 4-quinolinium | H | Me | -SMe | cyclohexyl | H | H | 0 |
| 823 | S | 4-quinolinium | H | Me | Cl | phenyl | H | H | 1 |
| 830 | S | 4-quinolinium | H | Me | Cl | thienyl | H | H | 0 |
| 834 | S | 4-quinolinium | H | Me | F | phenyl | H | H | 0 |
| 835 | S | 4-quinolinium | H | Me | -O-phenyl | phenyl | H | H | 0 |
| 853 | S | 4-quinolinium | H | Me | -S-2-pyridyl | phenyl | H | H | 0 |
| 854 | S | 4-quinolinium | H | Me | -OSO₂CF₃ | phenyl | H | H | 0 |
| 856 | S | 4-quinolinium | H | Me | N-Me-piperazyl | phenyl | H | H | 0 |
| 5103 | S | 4-quinolinium | H | Me | Cl | phenyl | H | -OMe | 0 |
| 6104 | S | 4-quinolinium | H | Me | cyclohexyl | Me | H | H | 0 |

### Synthesis

In general, synthesis of these dyes requires three precursors: a benzazolium salt, a pyridinium (or quinolinium) salt (both of which have the appropriate chemical substituents), and (where n = 1 or 2) a source for the methine spacer. Although the combination that enables these compounds to be useful stains for nucleic acids has not been described previously, the chemisty that is required to prepare and combine these precursors so as to yield any of the subject derivatives is generally well-understood by one skilled in the art.

### The benzazolium moiety.

A wide variety of derivatives of this type have been described (Brooker, et al., J. AM. CHEM. SOC., **64,** 199 (1942)) and Hamer, "The Cyanine Dyes and Related Compounds", THE CHEMISTRY OF HETEROCYCLIC COMPOUNDS, Vol. 18, A. Weissberger, Ed., Interscience, New York (1964). These precursors have the common structure:

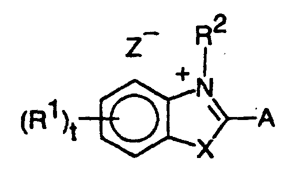

X may be O (benzoxazolium), S (benzothiazolium), Se (benzoselenazolium), N or an alkyl-substituted N (benzimidazolium) or a carbon atom substituted by two alkyl groups R¹⁶R¹⁷ (indolium) (where R¹⁶ and R¹⁷ are independently alkyl groups having 1-6 carbons, or R¹⁶ and R¹⁷ taken in combination complete a five or six membered saturated ring).

R¹ is usually incorporated in the parent benzazole molecule prior to quaternization with an alkylating agent. R² is usually obtained by alkylation of the parent heterocycle with R²-Z, where R² is an alkyl group having 1-6 carbons and Z is an electronegative group that frequently becomes the counterion on the resultant dye. Z⁻ is a biologically compatible counterion that additionally is stable and synthetically accessible. The counterion may be exchanged for another counterion by methods known in the art, such as the use of ion exchange resins or by precipitation. Preferred R²-Z are compounds that yield R² = methyl, such as methyl iodide.

A is a substituent whose nature is determined by the synthetic method utilized to couple the benzazolium precursor with the pyridinium or quinolinium precursor. When n = 0, A is usually alkylthio, commonly methylthio, or A is chloro, bromo or iodo. When n = 1 or 2, A is methyl.

### The pyridinium or quinolinium moiety.

The second heterocyclic precursor is a pyridinium or quinolinium sail These can sometimes be generated from the corresponding pyridine or quinoline by alkylation at nitrogen using a suitable alkylating agent R⁵-Z. However, 2- and 4-pyridones and 2- and 4-quinolones are much more versatile chemical intermediates, with the added advantage of being easily prepared (for examples see HETEROCYCLIC COMPOUNDS. VOL 4, R. C. Elderfield ed., John Wiley and Sons Inc., (1952) pp 1-331 or Wawzonek et al., J. HETEROCYCLIC CHEM., 25, 381 (1988)).

Typically the required pyridinium salt precursor has the structure 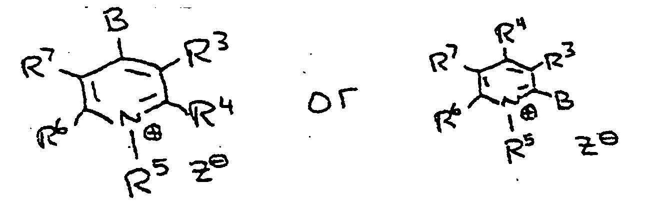 and the quinolinium salt precursor has the structure 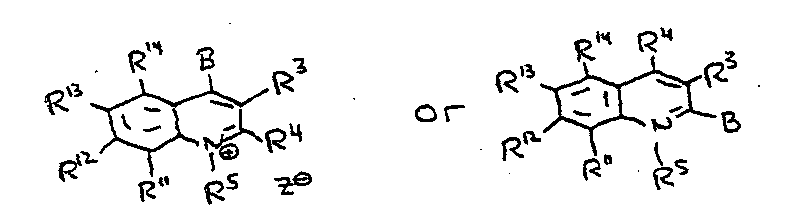 At all times, the ring is a cationic 6-membered pyridinium- or quinolinium-based heterocycle.

When n = 0, B is methyl, or B is chloro, bromo or iodo. When n = 1 or 2, B is methyl. When n = 1 or n = 2 part of B can be incorporated in the final compound.

When R⁵ is an OMEGA or alkyl, the 2-pyridone or 4-pyridone or 2-quinolone or 4-quinolone can be treated with a powerful nucleophile such as a Grignard or an alkyl lithium reagent (Example 11) or with a metal hydride (Example 12), to generate the pyridinium or quinolinium salt after acid-catalyzed dehydroxylation.

The pyridone or quinolone can also be converted to a pyridinium or quinolinium salt by using an agent such as phosphorous oxychloride, phosphorous tribromide, diethylaminosulfur trifluoride (Example 5) or trifluoromethanesulfonic anhydride. The resulting activated intermediate can be condensed with the appropriate benzazolium salt to form the dye directly (Example 6) or the activated intermediate can be treated with alcohols, phenols, or alkoxides to yield ether derivatives (Example 10), thiols or thiolphenols to yield thioether derivatives (Example 8) or ammonia or amines to yield substituted or unsubstituted amino derivatives (Example 7).

### The methine bridge.

The methine bridge consists of 1, 3 or 5 methine (-CH=) groups that bridge the benzazolium rings and the pyridinium or quinolinium ring(s) in such a way as to permit extensive electronic conjugation.

Synthesis of monomethine dyes (n = 0) commonly uses a combination of reagents where the methine carbon atom results from either A on the 2-position of the benzazolium salt or B on the 2- or 4-position of the pyridinium or quinolinium salt being methyl and the other of A or B being a reactive "leaving group" that is typically methylthio or chloro (Brooker et al., supra).

To synthesize trimethine dyes (n = 1) or pentamethine dyes (n = 2) both A and B are methyl. In these cases the additional methine carbon of is provided by a reagent such as N-methylformanilide or ethyl orthoformate (HOUBEN-WEYL, supra) or the additional trimethine fragment is provided by a malonaldehyde equivalent such as 1,1,3,3-tetramethoxypropane: 1,1,3-trimethoxypropene, 3-(N-methylanilino)propenal or 1-anilino-3-phenylimino-1-propene (Sprague, supra).

### Subsequent modification of dyes

As described earlier, the reactivity of the 2-halogenated pyridinium or quinolinium intermediate offers a variety of synthetic methods for attachment of various substituents at the 2-position. However, the reactivity of the 2-halo derivatives is preserved even after conjugation with the benzazolium precursor, enabling conversion of the resulting dye in which R⁴ is halogen into the appropriate ether, amine and thioether analogs. as described above for the pyridinium and quinolinium precursors (Examples 7, 8 and 10).

### Method of Use

The use of the invention comprises combining a dye of the present invention with a sample that contains or is thought to contain a nucleic acid, incubating the sample for a time sufficient to obtain a detectable fluorescent response, and observing the fluorescent response. The sample is optionally combined with one or more additional dyes (preferably fluorescent dyes) having a response detectably different from that of the subject dyes.

Typically, the subject dye is present as a staining solution, which is prepared by addition of the dye to an aqueous solution that is biologically compatible with the sample. The staining solution is made by dissolving the dye directly in an aqueous solvent such as water, a buffer solution, such as buffered saline (preferably non-phosphate), or an organic water-miscible solvent such as dimethylsulfoxide (DMSO), dimethylformamide (DMF), or a lower alcohol such as methanol or ethanol, or acetonitrile. Typically the dye is preliminarily dissolved in an organic solvent (preferably 100% DMSO) at a concentration of greater than about 100-times that used in the staining solution, then diluted one or more times with an aqueous solvent such as water or buffer, such that the dye is present in an effective amount. An effective amount of dye is the amount sufficient to give a detectable fluorescent response when in the presence of nucleic acids. Typically staining solutions for cellular samples have a dye concentration greater than about 0.1 nM, and less than about 100 µM, more typically greater than about 1 nM. Staining solutions for electrophoretic gels typically have a dye concentration of greater than about 1 µM and less than about 10 µM, more typically about 4-5 µM. Staining solution for detection of free nucleic acids in solution typically have a concentration 10 nM-1 µM. The specific concentration of the staining solution is determined by the physical nature of the sample, and the nature of the analysis being performed, and can be optimized according to standard procedures such as described for cell samples in Example 16.

The dye is combined with a sample that contains a nucleic acid. The nucleic acid in the sample may be RNA or DNA, or a mixture thereof. Any DNA is optionally single-, double-, triple-, or quadruple-stranded DNA. The nucleic acid may be natural (biological in origin) or synthetic (prepared artificially). The nucleic acid may be present as nucleic acid fragments, oligonucleotides, or nucleic acid polymers, and may contain unnatural bases. The nucleic acid may be present in a condensed phase, such as a chromosome. The presence of the nucleic acid in the sample may be due to a successful or unsuccessful experimental methodology, undesirable contamination, or a disease state. Nucleic acid may be present in all, or only part, of a sample, and the presence of nucleic acids may be used to distinguish between individual samples, or to differentiate a portion or region within a single sample.

The nucleic acid may be enclosed in a biological structure, for example contained within a viral particle, an organelle, or within a cell. Cell types include, but are not limited to, eukaryotes, such as nucleated plant and animal cells, and prokaryotes, such as bacteria (including both Gram-negative and Gram-positive bacteria such as *Bacillus cereus*, *Bacillus subtilus*, *Clostridium sporogenes*, *Corynebacterium xerosis*, *Micrococcus luteus*, *Mycobacterium phlei*, *Propionibacterium freunderreichii*, *Staphylococcus aureus, Streptococcus pyogenes*, *Lactobacillus acidophilus*, *Cytophaga psychrophila*, *Enterobacter acrogenes*, *Escherichia coli*, *Flavobacterium meningosepticum*, *Klebsiella pneumonia*, *Neisseria subflava*, *Pseudomonas aeruginosa*, *Rhizobium trifolii*, *Salmonella oranienburg*, *Shigella sonnei, Vibrio parahaemolyticus* or combinations thereof), as well as yeast and other fungi, mycobacteria and mycoplasma. The nucleic acids enclosed in biological structures may be obtained from a wide variety of sources, including unfiltered or separated biological fluids (such as urine, cerebrospinal fluid, blood, lymph fluids, tissue homogenate, mucous, saliva, stool, or physiological secretions or other similar fluids); environmental samples such as soil, water and air, a fermentation medium such as from a biological reactor or food fermentation process such as brewing; or surface washes of materials, (e.g. food) or small amounts of solids such as retentates, scrapes, and smears; or liquid growth medium in which cells have been introduced for culturing. The cells are optionally discrete or individual cells, including microorganisms, or multiple cells associated with other cells in two or three dimensional layers, including multicellular organisms, embryos, tissues, biopsies, filaments, biofilms, etc. The nucleic acid may be endogenous or introduced as foreign material, such as by infection or by transfection. The cells may be viable or dead cells or a mixture thereof. The nearly universal permeability of the instant dyes, their accelerated rate of uptake and the low toxicity of the dyes to living systems enable the examination of nucleic acids in living samples with little or no perturbation caused by the dye itself. The dyes can also be used for staining nucleic acids in a cell or cells fixed and treated with routine histochemical or cytochemical procedures.

Alternatively, the nucleic acid, in any of the forms described previously, is not enclosed within a biological structure, but is present as a sample solution. The sample solution can vary from one of purified oligonucleotides or nucleic acids to crude mixtures such as cell extracts, biological fluids and environmental samples from the sources listed above. In some cases it is desirable to separate the nucleic acids from a mixture of biomolecules or fluids in the solution prior to combination with the dye. Numerous techniques exist for separation and purification of nucleic acids from generally crude mixtures with other proteins or other biological molecules. These include such means as electrophoretic techniques and chromatographic techniques using a variety of supports. When used for poststaining electrophoresis gels, the high sensitivity of the dyes of the present invention allow the detection of previously unmeasureable amounts of nucleic acids without requiring destaining. One embodiment of the invention, when used in conjunction with an ultraviolet transilluminator, allows detection of as little as 20 picograms of double-stranded DNA per band.

The sample may be combined with the staining solution by any means that facilitates contact between the dye and the nucleic acid. The contact can occur through simple mixing, as in the case where the sample is a solution. The dye may be added to the nucleic acid solution directly or may contact the solution on an inert matrix such as a blot or gel, a testing strip, or any other solid or semi-solid surface, for example where only a simple and visible demonstration of the presence of nucleic acids is desired. Any inert matrix used to separate the sample can be used to detect the presence of nucleic acids by observing the fluorescent response on the inert matrix. While the subject dyes have shown an ability to permeate cellular membranes rapidly and completely upon addition of the dye solution, any other technique that is suitable for transporting the dye across cell membranes with minimal disruption of the viability of the cell and integrity of cell membranes is also a valid method of combining the sample with the subject dye. Examples of suitable processes include action of chemical agents such as detergents, enzymes or adenosine triphosphate; receptor- or transport protein-mediated uptake; pore-forming proteins; microinjection; electroporation; hypoosmotic shock; or minimal physical disruption such as scrape loading or bombardment with solid particles coated with or in the presence of the dyes.

The sample is incubated in the presence of the dye for a time sufficient to form the fluorescent nucleic acid-dye complex. Detectable fluorescence in a solution of nucleic acids is essentially instantaneous. Detectable fluorescence within cell membranes requires the permeation of the dye into the cell. Preferably, the dye is added at a temperature optimal for normal activity of the cells within the operating parameters of the dyes (between about 5 °C and about 50 °C); typically this is room temperature (23 °C). At temperatures between 5-45 °C, visibly detectable fluorescence is obtained within about 15-20 minutes of combination with the sample, commonly within about 5 minutes. Preferred embodiments give detectable fluorescence inside cells in less than about 2 minutes. Lymphocytes loaded with 5 µM dye solutions give a fluorescent response in less than 5 seconds, too fast to measure by conventional fluorometry. This property is useful for observing nuclear structure and rearrangement, for example such as occurs during mitosis or apoptosis. While permeation and fluorescence is rapid for all embodiments, optimal permeation of the dye or formation of the nucleic acid complex is dependent upon the physical and chemical nature of the individual sample and the sample medium, and can be determined according to standard procedures such as described in Example 17.

The subject dyes bind non-covalently with nucleic acids to yield enhanced fluorescence, the level of enhancement being generally about 100-1000 fold, typically greater than about 300-fold (Table 2). These dyes generally exhibit improved quantum yields upon binding to nucleic acids, relative to Thiazole Orange, which translate directly into improved sensitivity in nucleic acid detection. While not every dye shows an improved quantum yield, other attributes of the subject dyes represent significant improvement, including enhanced permeation, enhanced rate of permeation, and/or the selectivity of excitation and emission bands to suit specific instrumentation.

To facilitate the detection of the nucleic acid-dye complex, the excitation or emission properties of the fluorescent complex are utilized. For example, the sample is excited by a light source capable of producing light at or near the wavelength of maximum absorption of the fluorescent complex, such as an ultraviolet or visible tamp, an arc lamp, a laser, or even sunlight. Preferably the fluorescent complex is excited at a wavelength equal to or greater than about 300 nm, more preferably equal to or greater than about 340 nm. The equipment commonly available for excitation of samples near 254 nm, between 300 and 310 nm, and near 365 nm can be used to excite any of the dyes of the present invention. Excitation by a source more appropriate to the maximum absorption band of the nucleic acid-dye complex, such as the 488 nm band of the argon laser, results in even higher sensitivity. Some examples permit excitation beyond 600 nm.

The fluorescence of the complex is detected qualitatively or quantitatively by detection of the resultant light emission at a wavelength of greater than about 400 nm, preferably greater than about 480 nm, more preferably at greater than about 500 nm. The emission is detected by means that include visible inspection, photographic film, or the use of current instrumentation such as fluorometers, quantum counters, plate readers, epifluorescence microscopes, and flow cytometers, or by means for amplifying the signal such as a photomultiplier. The nucleic acid concentration in a sample can also be quantified, as the fluorescence of the nucleic acid-dye complex is linearly dependent on concentration (Examples 22-23).

The wavelengths of the excitation and emission bands of the dyes vary with dye composition to encompass a wide range of illumination and detection bands (e.g. Table 3). This allows the selection of individual dyes for use with a specific excitation source or detection filter. In particular, dyes can be selected that match their excitation band with the commonly used argon laser, or emission bands that match preexisting filters such as a typical fluorescein long-pass set or multi-band set with fluorescein excitation and emission bands.

In addition, the dye can be selected to give a detection response that is different from that of other dyes desired to be used in combination with the subject dyes. Preferably the additional dye or dyes are fluorescent, for which the response to illumination that is detectably different from that of the subject cyclic-substituted unsymmetrical cyanine dyes. Any fluorescence detection system can be used to detect the difference in spectral characteristics between dyes. Preferably the dyes have the same or overlapping excitation spectra, but possess visibly different emission spectra, generally having emission maxima separated by >10 nm, preferably >20 nm, more preferably >50 nm.

The additional dyes are optionally used to differentiate cells or cell-free samples containing nucleic acids according to size, shape, metabolic state, physiological condition, genotype, or other biological parameters or combinations thereof. In one aspect of the invention, the additional dye or dyes are metabolized intracellularly to give a fluorescent product inside certain cells but not inside other cells, so that the fluorescent response of the cyclic-substituted unsymmetrical cyanine dye predominates only where such metabolic process is not taking place. Alternatively, the additional dye or dyes are specific for some external component of the -cell such as cell surface proteins or receptors. In yet another aspect of the invention, the additional dye or dyes actively or passively cross the cell membrane and are used to indicate the integrity or functioning of the cell membrane.

The additional dyes are added to the sample being analyzed to be present in an effective amount, with the optimal concentration of dye determined according to the cell density as above. Typically the concentration of each dye is between about 0.01 µM and about 100 µM. more typically between 0.1 µM and 10 µM. Each dye is optionally prepared in a separate solution or combined in one solution. Generally the dyes are present in the staining solution within about a five-fold molar range. but the molar ratio one to the other in the sample can vary from about 1:1 to about 1:100, and may vary depending on whether the dyes are added to the sample simultaneously or sequentially. After illumination of the dyed cells at a suitable wavelength, as above, the cells are analyzed according to their fluorescent response to the illumination. In addition, the differential fluorescent response can be used as a basis for sorting the cells or nucleic acids for further analysis or experimentation. For example, all cells that "survive" a certain procedure are sorted, or all cells of a certain type in a sample are sorted. The cells can be sorted manually or using an automated technique such as flow cytometry according to the procedures known in the art such as in US-A-4,665,024 to Mansour, et al. (1987).

In one embodiment of the invention, the subject dyes are used in combination with a second fluorescent dye (Dye II) to distinguish viable cells from dead cells, where Dye II is selective either for viable or for dead cells. Accordingly, the invention includes methods of staining nucleic acids in a sample wherein the nucleic acids are enclosed in a biological structure and the method further comprises combining the sample with one or more additional dyes, singly or in combination; where at least one additional dye is a fluorescent nucleic acid stain that is permeant or impermeant to cells; another additional dye is selectively permeant to Gram positive or Gram negative bacteria; and where each of the additional dyes has a fluorescent response to illumination that is detectably different from that of the other dyes. In one class of such methods, the nucleic acid stain is impermeant to cells with intact membranes. In another class of such methods, the nucleic acid stain is impermeant to cells with intact membranes and is a phenanthridium monomer or dimer derivative that is an ethidium, ethidium dimer, propidium; or is a benzazolium monomer or dimer derivative such as TOTO™, YOYO™, POPO™, BOBO™, TOPRO™, YO-PRO™, BO-PRO™, PO-PRO™. In a further class of such methods, the nucleic acid stain is permeant to cells or is selectively permeant to Gram-positive bacteria and is a C₄-C₈ alkyl-substituted phenanthridium, or 2'-(4-hydroxyphenyl)-5-(4-methyl-1-piperazinyl)-2,5'-bi-1H-benzimidazole, 2'-(4-ethoxyphenyl)-5-(4-methyl-1-piperazinyl)-2,5'-bi-1H-benzimidazole or 4', 6-diamidino-2-phenylindole dihydrochloride.

Considering now in more detail methods in which a Dye II is used, in one aspect of the invention, Dye II gives a detectable fluorescent response only in viable cells, such as fluorescent enzyme substrates and reagents described in Haugland, HANDBOOK OF FLUORESCENT PROBES AND RESEARCH CHEMICALS (1992-94) to selectively stain viable cells, including haloalkyl esterase substrates and calcein AM. Alternatively, Dye II gives a detectable fluorescent response only in dead cells, such as an impermeant dye that only becomes fluorescent upon passing through the cell membrane to bind to some intracellular component, such as an intracellular protein or nucleic acid. While there is not an exact equivalence between an intact cell membrane and the term "viability" (technically defined as the ability of a cell to maintain its existence), it is common to refer to cells where the cell membrane has been irreversibly disrupted as "dead" cells or "non-viable" cells. Suitable dyes include impermeant phenanthridium or benzazolium derivatives, including monomers or dimers thereof, such as ethidium homodimer, ethidium bromide, propidium iodide, TOTO™, BOBO™, POPO™, YOYO™, TO-PRO™, BO-PRO™, PO-PRO™ and YO-PRO™ (Molecular Probes) that give an enhanced fluorescence when complexed to intracellular nucleic acids. Loading times for impermeant Dye II dyes, such as phenanthridium or benzazolium dyes, is generally the same as previously discussed above. Cell permeant Dye II dyes selective for viable cells generally require longer loading times, particularly if such dyes require intracellular activity to generate fluorescence.

In cells for which Dye II is selective, both dyes are present because the subject dyes stain all cells, including those for which Dye II is selective. In the cells for which Dye II is selective (and both dyes are present) the intracellular fluorescent response of Dye II is optionally the same as the fluorescent response of Dye II alone (e.g. where Dye II effectively competes for nucleic acid binding relative to the subject dye) or is a response indicative of the presence of both dyes (as is the case where the competitive binding is less effective or where Dye II is not a nucleic acid stain). The fluorescent response of the subject dye alone is indicative of cells for which Dye II is not selective, either viable or non-viable cells as the case may be. The cells for which Dye II is selective are optionally sorted or counted, as above.

In another embodiment of the invention, the sample is combined with multiple fluorescent dyes to determine identification, and optionally viability. The additional fluorescent dye(s) binds selectively to cell surface components or is selectively permeant to certain cell types, and can be used in combination with Dye II to also indicate viability. The surface label that only stains externally is distinguishable from the dyes that stain intracellularly. When a surface dye is used in combination with one or more intracellular stains such as nucleic acid stains, a "bullseye" pattern of staining is seen -- i.e. a brightly stained interior within an exterior ringstain. Preferably, the surface label also has an emission spectrum that is detectably different from that of the other dyes used. Preferably the excitation spectrum of each dye or dye-nucleic acid complex overlaps the excitation spectrum of the other dye(s). More preferably, each dye complexed with nucleic acids has an excitation maximum between about 480 nm and 510 nm. Most preferably, each dye or dye-complex also excites in the UV between about 300 nm and 365 nm.

In one aspect of the invention, the appearance of the stained bacteria indicates the Gram reaction of the bacteria in the sample, and optionally whether or not the G⁺ or G⁻ bacteria present in the sample are viable. Gram positive (G⁺) bacteria are those that give a positive Gram stain, including but not limited to *Bacillus, Lactobacillus, Micrococcus, Streptococcus, Clostridium, Staphylococcus*, and *Mycobacterium,* among others. Gram negative (G⁻) bacteria are those that are negative for the Gram stain, including but not limited to *Escherichia, Enterobacter, Salmonella*, *Pseudomonas, Shigella*, *Klebsiella*, *Haemophilus*, *Neisseria*, *Proteus*, *Vibrio, Campylobacter*, and *Yersinia*, among others.

Preferably a cyclic-substituted unsymmetrical cyanine dye that (in combination with intracellular nucleic acids) gives a green or yellow-green fluorescence is used in combination with one or more of the following dyes: a) a C₄-C₈ alkyl substituted phenanthridium nucleic acid stain (preferably hexidium or C₆-substituted phenanthridium, Watkins, J.CHEM. SOC. 3059 (1952)) that selectively stains live G⁺ bacteria and all dead bacteria with an orange red fluorescent signal that partially or completely replaces the signal of the cyclic-substituted unsymmetrical cyanine dye; and/or b) a protein that is covalently bound to a fluorophore with a fluorescent response different from that of the phenanthridium dye in a) and from that of the cyclic-substituted unsymmetrical cyanine dye, preferably a lectin such as wheat germ agglutinin labeled with AMCA™ or Cascade Blue dye (Molecular Probes) that is selective for the cell surface of G⁺ bacteria, live or dead; and optionally c) a membrane impermeant benzazolium nucleic acid stain according to Dye II above, that has a fluorescent response different from that of the other dyes used, preferably dyes sold under the names TOTO™, YOYO™, BOBO™, POPO™, TO-PRO™, YO-PRO™, BO-PRO™, PO-PRO™ (Molecular Probes).

Table 5 summarizes the spectral response, where the cyclic-substituted unsymmetrical cyanine dye (I) has an emission maximum between 500 nm and 535 nm (e.g. dye 624); the phenanthridium dye (II) has an emission maximum between 580 nm and 650 nm (e.g. hexidium); the membrane impermeant benzazolium (III) nucleic acid complex has an emission maximum between 530 nm and 590 nm (e.g. TOTO™, YOYO™, TO-PRO™ or YO-PRO™); and the labeled protein (IV) has an emission maximum between 410 nm and 480 nm (e.g. AMCA™-or Cascade Blue-labeled wheat germ agglutinin). Careful matching of other fluorescent stains with equivalent selective permeability, excitation/emission spectra, and preferential binding affinity for nucleic acids allows substitution of the preferred combination of nucleic acid stains to discriminate between many different organisms, whether live or dead.

**Table 5**

| Panel # in Figure 1 | Dyes† | Live Gram (+) Bacteria | Live Gram (-) Bacteria | Dead Gram (+) Bacteria | Dead Gram (-) Bacteria |
|---|---|---|---|---|---|
| 1 | I | G | G | G | G |
| 2 | II | O | ― | O | O |
| 3 | III | ― | ― | Y | Y |
| 4 | IV | B | ― | B | ― |
| 5 | I, II | O | G | O | O |
| 6 | I, III | G | G | Y | Y |
| 7 | I, IV | G with B | G | G with B | G |
| 8 | II, III | O | ― | Y | Y |
| 9 | II, IV | O with B | ― | O with B | O |
| 10 | III, IV | B | ― | Y with B | Y |
| 11 | I, II, III | O | G | Y | Y |
| 12 | I, II, IV | O with B | G | O with B | O |
| 13 | I, III, IV | G with B | G | Y with B | Y |
| 14 | II, III, IV | O with B | ― | Y with B | Y |
| 15 | I, II, III, IV | O with B | G | Y with B | Y |
| Color Key: G = True-green Y = Yellow-green O = Orange B = Blue Halo ―=Unstained | | | | | |

The examples below are given so as to illustrate the practice of this invention. They are not intended to limit or define the entire scope of this invention. In the structural formulae below, the substituent phenyl is represented by the symbol Ø, as is generally used and understood in the art.

### Example 1: Preparation of 1,2-dihydro-4-methyl-1-phenyl-2-quinolone (1)

The following compound is prepared: 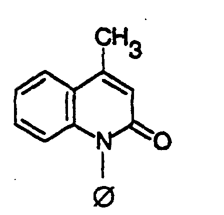 The synthetic precursor (1) is prepared either by an Ullmann coupling according to a literature procedure (Wawzonek, et al., supra.) or via the reaction of the corresponding diarylamine with diketene followed by acid cyclization (Elderfield, supra). Thus 10.0 g (62.9 mmoles) of 2-hydroxy-4-methylquinoline is heated at reflux with 24.0 g (377 mmoles) of copper powder, 8.68 g (62.9 mmoles) of potassium carbonate and 19.2 g (94 mmoles) of iodobenzene for 48 hours. The reaction is cooled to room temperature, partitioned between water and ethyl acetate, filtered, and the organic layer is dried over magnesium sulfate. The crude product is purified on a silica gel column, eluting with 1:1 ethyl acetate/hexanes to yield 8.1 g of the desired product

### Example 2: Preparation of 1,2-dihydro-4-methyl-1-phenyl-2-pyridone (2)

The following compound is prepared: 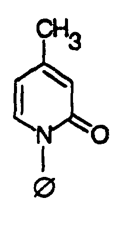 Synthetic precursor 2 is prepared as in Example 1 with a 40% yield, except that the starting material is 1,2-dihydro-4-methyl-2-pyridone.

### Example 3: Preparation of 1,2-dihydro-1,4-dimethyl-2-quinolone (3)

The following compound is prepared: 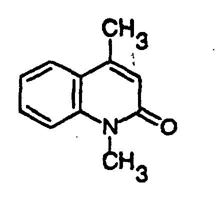 Synthetic precursor 3 is prepared by first conjugating N-methylaniline with diketene, followed by an acid cyclization of the amide intermediate. Thus 10.0 g (0.12 moles) of diketene is added dropwise to 10.7 g (0.1 moles) of N-methylaniline and the reaction is heated at 100 °C for an additional 30 minutes. To the resulting mixture is added 30 mL of acetic acid and 30 mL of sulfuric acid. and the mixture is heated at 50 °C overnight. The reaction is worked up with water and ethyl acetate and purified on a silica gel column to yield 9.5 g of the desired product.
If the synthesis is performed using N-methyl-2-phenylaniline (generated by methylation of 2-phenylaniline using K₂CO₃ and CH₃I) the resulting product is 1,2-dihydro-1,4-dimethyl-8-phenyl-2-quinolone.

### Example 4: Preparation of 1,2-dihydro-7-methoxy-4-methyl-1-phenyl-2-quinolone

The following compound is prepared: 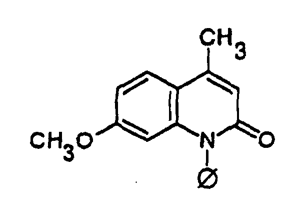 N-(3-hydroxyphenyl)-N-phenylamine is O-methylated with potassium carbonate and methyl iodide in acetone in 39% yield. The resulting N-(3-methoxyphenyl)-N-phenylamine is then reacted with diketene to generate the corresponding acetoacetamide which, without purification, is cyclized in acetic acid/sulfuric acid as in Example 3 to generate the desired quinolone in 41% yield.

### Example 5: Preparation of 2-chloro-4-methyl-1-phenylquinolinium chloride (4)

The following compound is prepared: 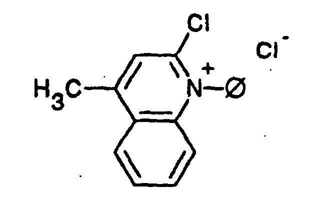 To 2.8 g (11.9 mmoles) of **1** in 20 mL of methylene chloride is added 1.85 g of POCl₃ and a catalytic amount of dimethylformamide (Marson, TETRAHEDRON., **48**, 3659 (1992)). The resulting mixture is heated to reflux for 24 hours. Alter cooling, the product is purified using column chromatography.
The corresponding bromide is prepared using PBr₃ rather than POCl₃.
The corresponding fluoride is prepared using diethylaminosulfur trifluoride, rather than POCl₃.

### Example 6: Preparation of 2-chloro-4-[2,3-dihydro-3-methyl-(benzo-1,3-thiazol-2-yl)-methylidene]-1-phenylquinolinium iodide (dye 591)

The following compound is prepared: 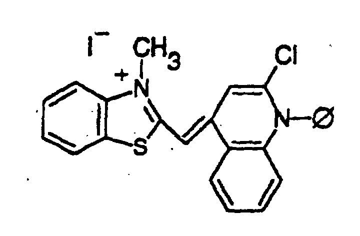 A room temperature solution of 4 (11.9 mmoles) is prepared, and 3.5 g (9.6 mmoles) of N-methyl-2-methylthiobenzothiazolium tosylate (5) (Rye, et al., NUCLEIC ACIDS RES., **20**, 2803 (1992)) is added followed by 1.3 mL (9.4 mmoles) of triethylamine. The mixture is stirred for an additional 6 hours. The crude product is purified on silica gel using ethyl acetate:chloroform:methanol, 3:3:1 as eluant. The product is then recrystallized from methanol/chloroform/ethyl acetate.
The corresponding bromide (Dye 774) is prepared analogously using 2-bromo-4-methyl-1-phenylquinolinium bromide in place of 4.
The corresponding fluoride (Dye 834) is prepared analogously using 2-fluoro-4-methyl-1-phenylquinolinium fluoride in place of 4.
The methoxyquinolinium analog (Dye 5103) is prepared in the same way, except using 1,2-dihydro-7-methoxy-3-methyl-1-phenyl-2-quinolone.
The pyridinium analog (Dye 678) is prepared in the same way, except using the pyridinium analog of 4.
The trimethine dye analog (Dye 823) is prepared similarly, except using 2-(2-anilinovinyl)-3-methylbenzothiazolium tosylate in place of 5.
An additional synthetic route to Dye 591 utilizes 4-[2,3-dihydro-3-methyl-(benzo-1,3-thiazol-2-yl)-methylidene]-1,2-dihydro-1-phenyl-2-quinolone (6), which in turn is prepared from 1 and 5. Thus the lithium enolate of 1 (prepared from treating the quinolone with 2.7 equivalent of lithium diisopropyl amide) or the silyl enolate of 1 (from (1) and trimethylsilyl trifluoromethanesulfonate and diisopropylethylamine) is stirred with 5. The desired intermediate (6) is isolated by column chromatography. The quinolone (6) is then treated with POCl₃ to generate Dye 591.

### Example 7: Preparation of 2-diethylamino-4-[2,3-dihydro-3-methyl-(benzo-1,3-thiazol-2-yl)-methylidene]-1-phenylquinolinium iodide (Dye 619)

The following compound is prepared: 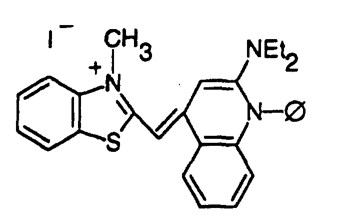 Dye 619 is prepared by heating Dye 591 (26 mg) at 55 °C with 0.5 mL of diethylamine in 1.5 mL of DMF overnight. The desired product is isolated by a simple filtration.
Dye 752 is prepared similarly, except using morpholine in place of diethylamine in DMF at 50 °C.
Dye 856 is prepared similarly, except using N-methylpiperazine in place of diethylamine.
Dye 130 is prepared similarly, except using aniline in place of diethylamine.
2-(N-3-dimethylaminopropyl)-N-propylamino-4-[2,3-dihydro-3-methyl-(benzo-1,3-thiazol-2-yl)-methylidene]-1-phenylquinolinium ioide (Dye 1037) is prepared similarly, except using N-(3-dimethylaminopropyl)-N-propylamine in place of diethylamine.

### Example 8: Preparation of 4-[2,3-dihydro-4-methyl-(benzo-1,3-thiazol-2-yl)-methylidene]-1-phenyl-2-(2-pyridylthio)-quinolinium iodide (dye 853)

The following compound is prepared: 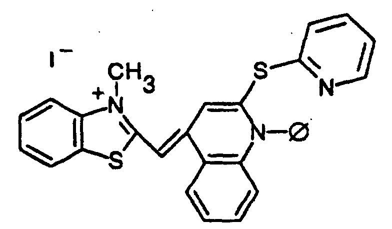 2-Mercaptopyridine (6.3 mg) is added to 25 mg of Dye 591 in 2 mL of methylene chloride, followed by 13 µL of triethylamine, and the resulting mixture is stirred at room temperature for 1.5 hours. The volume of solvent is reduced to about 0.5 mL under reduced pressure and the product is isolated by filtration.
2-(2-Dimethylaminoethylthio)-4-[2,3-dihydro-3-methyl-(benzo-1,3-thiazol-2-yl)-methylidene]-1-phenylquinolinium iodide (Dye 1004) is prepared analogously, using Dye 633 in place of Dye 591, and 2-dimethylaminoethanethiol in place of 2-mercaptopyridine.

### Example 9: Preparation of 2-chloro-4-[2,3-dihydro-3-methyl-(benzo-1,3-thiazol-2-yl)-methylidene]-1-cyclohexylquinolinium tosylate (Dye 780 (CI))

The following compound is prepared: 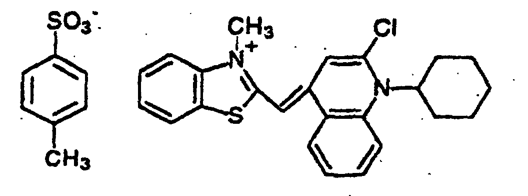 1-Cyclohexyl-1,2-dihydro-4-methyl-2-quinolone is prepared using N-cyclohexylaniline as starting material. The quinolone (0.482 g, 2 mmol) is transformed to the 2-chloro-1-cyclohexylquinolinium chloride with a procedure similar to Example 5, and is then reacted with 5 (0.74 g. 2 mmol) and triethylamine (0.28 mL, 2 mmol) to yield the product.

### Example 10: Preparation of 2-methoxy-4-[2,3-dihydro-3-methyl-(benzo-1,3-thiazol-2-yl)-methylidene-1-phenylquinolinium iodide (dye 628)

The following compound is prepared: 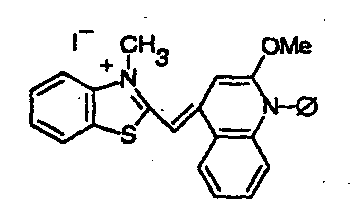 The intermediate compound (4) (4.3 mmoles) and methanol (10 mL) are heated to reflux for 2 hours. The methanol is removed under reduced pressure, and 10 mL of methylene chloride is added, followed by 1.56 g (4.3 mmoles) of 5 and 1.5 mL of triethylamine. The resulting mixture is stirred at room temperature for 3 days. The crude material is purified on a silica gel column by eluting with 5:5:1 ethyl acetate: chloroform: methanol.
Thc corresponding ethoxide (Dye 715) is prepared analogously, using ethanol rather than methanol.

### Example 11: Preparation of 2-butyl-4-[2,3-dihydro-3-methyl-(benzo-1,3-thiazol-2-yl)-methylidene-1-phenylquinolinium iodide (dye 61)

The following compound is prepared: 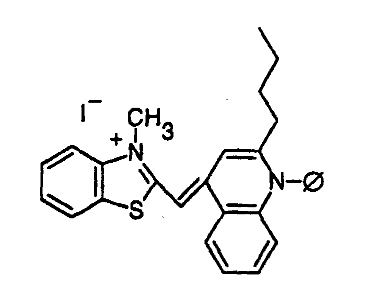 To 0.235 g (1 mmole) of 1 in 10 mL of THF at -78 °C under nitrogen, 1.2 equivalents of n-butyl lithium is introduced. The reaction is stirred at -78 °C for 15 minutes, and then the temperature is raised to 0°C for another 30 minutes, then the reaction is quenched with acetic acid and the solvent is evaporated. The residue is dissolved in 5 mL of methylene chloride and 0.367 (1 mmole) of 5 is added followed by 0.28 mL (2 mmoles) of triethylamine. The reaction mixture is stirred for 20 minutes at room temperature and the crude product is isolated as the iodide salt after a salt exchange. The crude iodide is recrystallized from methanol.
Dye 624 is prepared similarly, except that 3-methyl-2-methylthiobenzoxazolium tosylate (7) (Rye, et al., *supra*) is used instead of 5 in the synthesis.
Dye 6104 is prepared similarly, except that cyclohexyl magnesium bromide is used instead of butyl lithium.
The corresponding trimethine dye (Dye 621) is prepared similarly, except that 2-(2-anilinovinyl)-3-methylbenzothiazolium tosylate is used in place of 5.
The corresponding pentamethine dye (Dye 100) is prepared similarly, except that 2-(4-anilino)-1,3-butadienyl)-benzothiazolium iodide is used in place of 5. 2-(4-Anilino)-1,3-butadienyl)-benzothiazolium iodide is prepared using methods known in the art (U.S. Patent No. 2,269,234 to Sprague (1942); and HOUBEN-WEYL METHODON DER ORGANISCHEN CHEMIE, Band V/1d, 231-299 (1972)) from 1,3-dimethylbenzothiazolium iodide and 1-anilino-3-phenylimino-1-propene hydrochloride.

### Example 12: Preparation of 4-[(2,3-dihydro-3-methyl-(benzo-1,3-thiazol-2-yl)-methylidene]-1-phenylpyridinium iodide (dye 640)

The following compound is prepared: 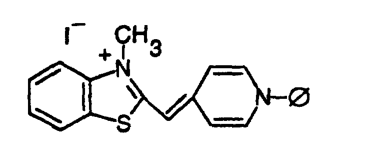 To 0.37 g (2 mmoles) of 1,2-dihydro-1-phenyl-2-pyridone in 10 mL of methylene chloride at 0 °C is added 2.2 mL of 1.0 M DIBAL (in cyclohexane) and the resulting mixture is stirred at a low temperature for 2 hours. Acetic acid (0.3 mL) is added, and the volatile components are evaporated. The residue is dried, then is redissolved in 15 mL of methylene chloride. 0.74 g (2 mmoles) of 5 is added followed by 0.28 mL (2 mmoles) of triethylamine. The reaction mixture is stirred at room temperature for 3 hours and the crude product is loaded on a silica gel column and eluted with 3:3:1 ethyl acetate/chloroform/methanol. The fractions containing the product are pooled and evaporated, redissolved in 5 mL of DMF and added to 5 g of sodium iodide in 75 mL of water. The precipitate is filtered and recrystallized from methanol.
If 1,2-dihydro-1,4-dimethyl-8-phenyl-2-quinolone is used in place of the pyridone, the reaction produces Dye 72.

### Example 13: Preparation of 4-[(2,3-dihydro-3-methyl-(benzo-1,3-thiazol-2-yl)-methylidene]-1-cyclohexylquinolinium iodide (dye 73)

The following compound is prepared: 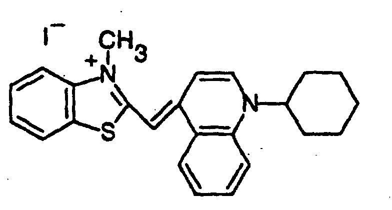 A mixture of 1.43 g (10 mmoles) of lepidine and 2.1 g (10 mmoles) of cyclohexyl iodide is heated at 130°C for 2 hours. Ethyl acetate (20 mL) is added and 1.36 g of solid is obtained after filtration. The solid is stirred in 50 nL of methylene chloride with 1.41 g of 5 and 1.12 mL of triethylamine for several hours. The crude product is converted to the iodide salt and recrystallized from methanol to yield the pure product.

### Example 14: Preparation of 2-[(2,3-dihydro-3-methyl-(benzo-1,3-thiazol-2-yl)-methylidene]-1-phenylquinolinium iodide (dye 64)

The following compound is prepared: 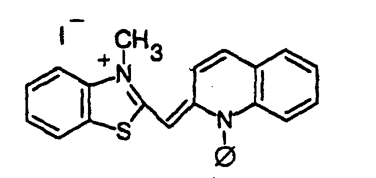 The intermediate N-phenyl-2-chloroquinolinium chloride is prepared according to Marson (TETRAHEDRON, **48**, 3659 (1992)). Thus 1.06 g (5 mmoles) of N,N-diphenylacetamide is heated with 1.69 g (11 mmoles) of POCl₃ and 0.44 g (6 mmoles) of DMF at 120 °C for 2 hours. The reaction mixture is cooled to room temperature and 15 mL of methylene chloride is added to dissolve the residue. To the solution is added 1.68 g (5 mmoles) of 2,3-dimethylbenzothiazolium tosylate and 1.46 g (12 mmoles) of 4-dimethylaminopyridine, and the reaction is stirred overnight (Elderfield, supra). The crude product is first purified on a silica gel column eluting with 2:2:1 ethyl acetate/chloroform/methanol and then metathesized to the iodide salt and recrystallized from methanol to obtain the pure product.

### Example 15: Preparation of 4-[2,3-dihydro-4-methyl-(benzo-1,3-thiazol-2-yl)-methylidene] -1-phenyl-2-trifluoromethanesulfonyloxyquinolinium iodide (dye 854)

The following compound is prepared: 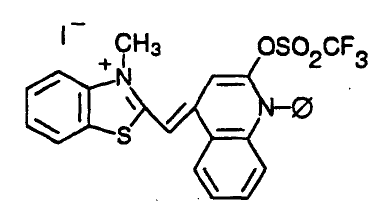 Trifluoromethanesulfonic acid anhydride (66 µL) is added to 0.1 g of 6 in 5 mL of 1,2-dichloroethane, and the solution is heated at 80 °C for 3 hours. The reaction is worked up with water and chloroform, and the resulting product is purified by chromatography on silica gel.

### Example 16: Optimization of Dye Loading

Cell density is determined by counting or by the following extrapolation. A cell culture is washed by centrifugation and resuspended in water to its original volume. Using flat-bottom 96-well microtiter plates, 150 µL volumes of suspension are loaded per well. A single well of sterile water is the well background standard. Using a Dynatech MR600 microplate reader equipped with a 410 nm filter, absorbance is determined for the initial volumes of suspension. The suspension is diluted seven times by serial ten-fold dilutions in water, 150 µL of suspension per well, and the absorbance measured for each dilution. Following the absorbance measurements, each dilution loaded into wells is further diluted 1:10 and plated in duplicate on nutrient growth agar. The colonies are counted and expressed as colony forming units per milliliter (cfu/mL). Using the turbidity of the dilution in the microtiter plate, the suspension is diluted to a density of about 1x10⁹ cfu/mL.
The cell suspension, adjusted to a known density, is diluted seven times by serial ten-fold dilutions in water; 150 µL of suspension per well. Three-fold serial dilutions of dye are used (30-0.04 µM); 50 µL of dye at 4x final concentration. Using 96-well flat-bottom plates, a matrix is set up whereby the cell concentration decreases across the plate and the dye concentration decreases down the plate, final volume per well is 200 µL. The top row and first column are reserved for the control, sterile water. The plate is incubated at 37 °C for 30 minutes, then read in a CytoFluor™ 2350 fluorescence microplate reader at a fixed excitation of 485 +/-10 nm and each of three emission wavelengths, 530 +/-12, 620 +/-20, or 645 +/-20 nm. The results determine the best dye range (30-1 µM) and the best cell concentrations (concentrated through first three ten-fold dilutions) for optimal dye loading. These results lead to the next staining optimization assay. Using the four dye dilutions and the four cell dilutions, many cultures and dyes can be assayed quickly. The data collected allow the determination of optimal dye and cell concentration required for maximal fluorescence intensity per cell.

### Example 17: Rate of Dye Loading

Minimum times for dye loading are obtained as follows: Cells are grown in nutrient broth to log phase. washed by centrifugation, and resuspended in water to a density previously shown to allow dye loading to maximal fluorescence/cell. Fluorescence cuvettes containing the cell suspension are placed in a fluorescence spectrophotometer equipped with a temperature regulated cuvette holder and magnetic stirrer. The suspensions are brought to the appropriate temperature prior to dye addition. Millimolar dye stock solutions in DMSO are added at the appropriate concentrations to produce maximum attainable fluorescence/cell at the peak emission wavelength of each dye. Fluorescence intensity of the suspensions is measured at or near the peak excitation and emission wavelengths for the dye (see e.g. Table 3). Sampling of fluorescence is carried out until the fluorescence signal stabilizes. Comparison of loading times at 5 °C, 23 ° C, and 37 °C shows a marked enhancement of rate of loading as the temperature increases, after equilibrating the suspensions at the appropriate temperatures and adding the dye as described above.

### Example 18: Staining Motile Cells

A frozen suspension of goat sperm is thawed and held at 32 °C. Enough of a 10 mM dye stock solution (dye 628, 624, 835 or 591) is added to the sperm suspension to obtain a final concentration of 0.5 µM dye. The sperm are labeled by incubation in the dye solution for 10 min. Sperm cells stain with all of the dyes, and the order of brightness is 628 > 624 >> 835 > 591. Motility is retained at 0.5 µM, but is lost in some sperm at s µM dye.

### Example 19: Staining Tissue

A leaf of *Aucuba spp.* is cross-sectioned with a razor blade and immersed in 0.5 mL of a 10 µM solution of dye 624 in E-pure water in a 35 mm glass dish. The tissue is stained for 30 min at room temperature in the dark. The tissue preparation is mounted in the presence of dye between coverglass and slide. The leaf epidermal layer is demarcated by a large amount of yellow autofluorescence, however both the vascular bundle and cell nuclei stain bright green in the dye 624-loaded cells.

### Example 20: Staining Compartmentalized Nucleic Acids

A 10 mM stock solution of dye 613 is added to a suspension of Infectious Hepatic Necrosis Virus in 135 mM NaCl, 5 mM KCI, 1 mM MgCl₂, 1.8 mM CaCl₂, 20 mM Na-HEPES, at pH 7.4 (HBSS+) to give a 40 µM dye solution. After incubation for 10 minutes at 15 °C, the viruses are observed in an epifluorescence microscope using a 100x objective lens. The virus particles (~30 x 160 nm) are below the resolution limit of the microscope using visible light. Incorporation of dye 613 into the viral RNA results in a sufficient concentration of the dye in the particle to render it visible as a bright point of green light when observed using a standard fluorescein long-pass filter set.

### Example 21: Staining Organellar Nucleic Acids

3T3 mouse fibroblast cells are grown on coverslips in calf serum-supplemented Dulbecco's Modified Eagle medium. Coverslips of cells are washed using HBSS+, then incubated for 30 min at room temperature in solutions of dye 835 with final concentrations of either 2 µM or 0.2 µM prepared in HBSS+. Cells are then washed in HBSS+ and viewed by epifluorescence microscopy using a long-pass fluorescein filter set. After 30 minutes all of the cells are stained green in both the nucleus and cytoplasm, although to different intensities, when viewed through the long-pass fluorescein filter. Cells loaded with 0.2 µM dye show distinct mitochondrial staining whereas cytoplasmic fluorescence appears to be less punctate in cells incubated with 2 µM dye. Nuclear staining is fairly uniform and is not concentrated in the nucleolar regions. Cell viability, as determined using an ethidium homodimer counterstain, is maintained throughout.

### Example 22: Staining Cell-free Nucleic Acids

To quantify the amount of DNA or RNA in solution, dye 61 is prepared as 10 mM stock solution in DMSO, then diluted to 2 µM in TNE buffer (2 M NaCl, 10 mM Tris. 1 mM EDTA, adjusted to pH 7.4). Calf thymus DNA or yeast ribosomal RNA solutions between 1-40 µg/mL are prepared in TNE buffer and mixed 1:1 with diluted dye. Fluorescence of 100 µL samples is measured in a CytoFluor fluorescence microplate reader. A linear increase in fluorescence is obtained with increasing DNA or RNA concentration.

### Example 23: Quantitative Analysis Using a Fluorometer

The density of a suspension of *E.coli* is indicated by adding enough cells to cuvettes containing 30 µM dye 624 to effect final densities of 10⁵ -10⁸ bacteria/mL and incubating for 5 minutes. The suspensions are excited at 480 nm and the fluorescence emission spectra of the suspensions are measured in a fluorometer. The green fluorescence of the bacterial suspensions increases with decade changes in bacterial cell density.

### Example 24: Viability Analysis Using Visual Observation

Peripheral blood lymphocytes are isolated from whole goat blood using the standard ficoll density gradient protocol. The cells are incubated in saline buffer with coverslips coated with a cell adhesive. After attachment to the coverslip, the cells are incubated with either
a) 1 µM dye 637 for 30 minutes followed by washing, and subsequently incubating with 1 µM calcein AM for 30 minutes, or
b) as above but labeling first with calcein AM. and subsequently staining with dye 637.
After washing with saline, the stained cells are viewed through a long-pass fluorescein filter to view calcein fluorescence and a long-pass Texas Red® filter to view the emission of dye 637. The majority of cells are visible using both the red and green filters. Cells that are dead, however, are stained only with dye 637 and do not exhibit green fluorescence, regardless of order of staining.

### Example 25: Viability Analysis Using Flow Cytometry:

Cultures of either *E. coli* or *Staph*. *aureus* are grown to late log phase in 30 mL of nutrient broth. A 25 mL suspension of the culture is concentrated by centrifugation at 10,000 rpm for 10-15 minutes. The supernate is discarded and the pellet is resuspended by triturating in 2 mL sterile, filtered water. Two 30-40 mL centrifuge tubes are prepared containing, respectively, 20 mL sterile water (for the live bacteria standard) and 20 mL 70% isopropyl alcohol (for the dead bacteria standard). To each of the centrifuge tubes is added 1 mL of the resuspended bacterial sample. Both tubes are then incubated at room temperature for 1 hour, mixing every 15 minutes. Both samples are then centrifuged as above and washed. The pellets are then resuspended in separate tubes using 10 mL of sterile water in each tube. The optical density of each suspension is then determined at 670 nm. The optical density of the suspensions is then adjusted to 1 x 10⁸ bacteria/mL (0.03 OD₆₇₀) for *E. coli* or 1 x 10⁷ bacteria/mL (0.149 OD₆₇₀) for *Staph*. *aureus.* The 1 x 10⁸ bacteria/mL (0.03 OD₆₇₀) suspensions are then diluted 1:100 in sterile water to give a final bacterial density of 1 x 10⁶ bacteria/mL for both bacterial samples. Eleven different proportions of *E. coli* are prepared to yield live:dead ratios between 0 and 100% in 10% increments. The volume of each bacterial sample is 2 mL.
A staining solution is prepared that is 1.67 mM in dye 624 and 10 mM in propidium iodide. Each of the 11 samples is stained with 6 µL of the staining solution and mixed thoroughly. The samples are incubated in the dark for 15 minutes.
The bacterial samples are analyzed using a flow cytometer (Coulter Electronics, Hialeah, Florida) equipped with an argon laser (488 nm excitation), two photomultipliers (PMT), and a 76 µm flow tip. The emission light path contains a 515 nm blocking filter, 590 nm dichroic filter before the Green PMT, and a 610 nm absorbance filter before the Red PMT. The fluorescence acquisition is gated on the log integrated green fluorescence (LIGFL) and discriminated at the 15% level on LIGFL since both live and dead bacteria have a measurable green signal. The populations of bacteria are discriminated by the ratio of LIRFL to LIGFL and the numbers of bacteria found within these regions are used to determine the percentage of viable organisms in the population.

### Example 26: Viability Analysis Using a Microplate Reader:

Suspensions of live and dead *E. coli* or *Staph*. *aureus* are prepared as in Example 25, except that the *Staph*. *aureus* suspension is adjusted to an optical density of 5 x 10⁶ bacteria/mL (0.074 OD₆₇₀), and incremental mixtures of live and dead bacteria are likewise prepared. Sterile filtered water serves as a reagent blank.
A staining solution is prepared that is 1.67 mM in dye 624 and 10 mM in propidium iodide. To 6.6 mL of sterile water is added 40 µL of the staining solution, and the new solution is mixed thoroughly.
Into each test well of a 96-well flat-bottom microplate is pipetted 100 µL of the mixed live/dead bacterial suspensions. Using a new pipet tip for each row, 100 µL of the diluted staining solution is pipetted into each appropriate well in the row. The plate is then incubated in the dark for 15 minutes. The appropriate gain setting and filters are set on the specific fluorescence microplate reader. The excitation filter is set to 485 ± 20 nm (blue) and the emission filter is set to 530 ± 25 nm (1). The fluorescence emission intensity of the entire plate is measured, and the data saved. The emission filter is set to 620 ± 40 nm (2), retaining the blue excitation. The fluorescence emission intensity of the entire plate is measured, and the data saved. The fluorescence data are analyzed by subtracting the fluorescence of the reagent solution in water from the fluorescence of the stained cell suspensions with each filter combination and dividing the corrected fluorescence emission 1 by the fluorescence emission 2. The corrected ratio versus percent live bacteria suspension is plotted and used as a calibration curve for determining live/dead ratios in bacterial samples.

### Example 27: Cell Differentiation Using Flow Cytometry

Blood is collected aseptically in a K₃EDTA-containing tube and maintained at room temperature. 5 µL of whole blood is added to 1 mL of a 30-90 nM solution of dye 628 or 591 in 135 mM NaCl, 5 mM KCl, and 20 mM Na-HEPES, at pH 7.4 (HBSS-). The suspension is incubated at room temperature for between 10 min and 3 hr. The cells are analyzed in a flow cytometer by gating around the erythrocyte population. Fluorescence is excited at 488 nm and emission is measured between 520 and 550 nm. Cells with fluorescence above the autofluorescence of the erythrocyte population without dye are counted as reticulocytes. Reticulocyte staining of patient blood samples is compared with staining of reticulocyte standards (Retic Chex, Streck). Dyes 591 and 628 are effective stains for reliculocytes, both by the measurement of commercial reticulocyte standards and with populations of reticulocytes in normal blood and in blood from patients with hemolytic anemia.

### Example 28: Cell Differentiation Using Multiple Dyes

The Gram reaction and viability of the mixed bacterial suspension *of Staph*. *aureus* (5 x 10⁵/mL) and *E*. *coli* (1 x 10⁶/mL) in water is determined by automated fluorescence microscopy by loading the bacteria with 1 µM of TOTO-1 dye (Molecular Probes) in combination with 5 µM of dye 624 and 1 µM of hexidium bromide dye (C₆ alkyl substituted phenanthridium, Watkins, J.CHEM. SOC. 3059 (1952)). All dyes are prepared by dilution of 1 mM DMSO stock solutions in water. All dead bacteria appear very brightly fluorescent yellow-green, while live *S*. *aureus* bacteria appear orange-red and live *E. coli* appear green. (Figure 1& Table 5). Cell fragments that have no associated nucleic acids are not stained. Cells that are stained with a 2-fold lower concentration of the same dyes are analyzed using a flow cytometer equipped with a 488 nm Argon laser. The cells are sorted or counted based on red/green ratio and spectral intensity. Three populations are discerned.

### Example 29: Detection of Bacterial Contamination

Whole goat blood smears are prepared with 30 µL blood diluted 50:50 with HBSS-. Blood with or without 5 µL of *Mycobacterium phlei* (in 1% TX-100) per 100 µL of blood is used for the smears. Smears are air dried and heat fixed at 50 °C for 2 hours. 15 µL of 5 µM dye 628 in water are added to the smears. A coverslip is placed over the dye droplet and sealed. Bacteria are visible after < 30 sec. Numerous extremely bright bacteria can be seen in blood to which *Mycobacteria* have been added. Low background fluorescence is observed in blood without *Mycobacteria*, aside from a few tiny bright dots, which are much smaller than bacteria and not nearly as bright when observed by epifluorescence microscopy using a 40x or 100x objective lens.

It is to be understood that, while the foregoing invention has been described in detail by way of illustration and example, numerous modifications, substitutions, and alterations are possible without departing from the scope of the invention as described in the following claims.

## Claims

1. A compound of the formula 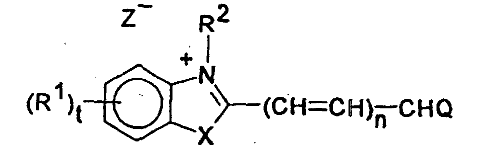 wherein
each R¹ is independently H; C₁-C₆ alkyl; trifluoromethyl; a halogen; or -OR⁸, -SR⁸ or -(NR⁸R⁹) where R⁸ and R⁹, which can be the same or different, are independently H, C₁-C₆ alkyl or 1-2 alicyclic, heteroalicyclic, aromatic or heteroaromatic rings, having a total of 3-16 ring atoms, wherein the hetero atoms are O, N or S, or R⁸ and R⁹ taken in combination are -(CH₂)₂-L-(CH₂)₂- where L = a single bond, -O-, CH₂-, or -NR¹⁰-, where R¹⁰ is H or C₁-C₆ alkyl;
t = 1-4;
R² is C₁-C₆ alkyl;
X is O or S;
n = 0, 1 or 2;
Z is a biologically compatible counterion;
Q has the formula Q1 or Q2 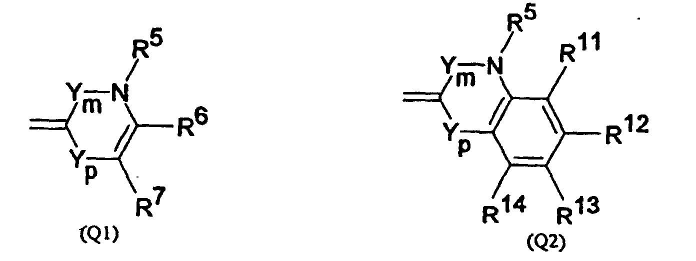
wherein
Y is -CR³=CR⁴-;
p and m = 0 or 1, such that p + m = 1;
R⁵ is C₁-C₆ alkyl or an OMEGA;
R³, R⁴, R⁶ and R⁷, which may be the same or different, are independently H, C₁-C₆ alkyl, a halogen, or -OSO₂R¹⁹ where R¹⁹ is alkyl having 1-6 carbons, or perfluoroalkyl having 1-6 carbons, or aryl; or an OMEGA; or -OH, -OR⁸, - SR⁸, -(NR⁸R⁹);
or R⁶ and R⁷, taken in combination are -(CH₂)ᵥ- where v = 3 or 4, or R⁶ and R⁷ form a fused aromatic ring according to formula Q2;
R¹¹, R¹², R¹³, and R¹⁴, which may be the same or different, are independently H; or an akyl group having 1-6 carbons; or a halogen; or an OMEGA; or -OH, -OR⁸, -SR⁸, or -(NR⁸R⁹);
OMEGA is cyclohexyl, cyclohexenyl, morpholino, piperidinyl, naphthyl, phenyl, thienyl, benzothiazolyl, furanyl, oxazolyl, benzoxazolyl or pyridinyl, any of the afore said OMEGA groups being unsubstituted or optionally substituted one or more times, independently, by halogen, alkyl, perfluoroalkyl, amino, alkylamino, dialkylamino, alkoxy or carboxyalkyl, having 1-6 carbons, and that is attached as R³, R⁴, R⁵, R⁶, R⁷, R¹¹, R¹², R¹³ or R¹⁴ by a single bond, and, where more than one of R³, R⁴, R⁵, R⁶, R⁷, R¹¹, R¹², R¹³ or R¹⁴ is an OMEGA, each OMEGA is the same or different;
provided that at least one of R³, R⁴, R⁵, R⁶, R⁷, R¹¹, R¹², R¹³ and R¹⁴ is an OMEGA and that when Q has the formula Q1, n = 0.

2. A compound according to claim 1 wherein m = 1 and R⁵ is an OMEGA.

3. A compound according to claim 1 or claim 2 wherein R⁴ is H; or R⁴ is an alkyl group having 1-6 carbons; or a halogen; or R⁴ is -OH, -OR⁸, -SR⁸, -(NR⁸R⁹); or R⁴ is-OSO₂R¹⁹; or R⁴ is an OMEGA.

4. A compound according to claim 3, wherein n = 0 or 1, and OMEGA is phenyl or substituted phenyl.

5. A compound according to claim 3 or claim 4, wherein
(i) R⁴ is halogen or -OSO₂R¹⁹; or
(ii) R⁴ is -SR⁸ or -(NR⁸R⁹).

6. A cyclic-substituted unsymmetical cyanine dye, comprising a first heterocyclic ring system that is a substituted benzothiazolium, benzoxazolium, benzoselenazolium, benzimidazolium, or dialkylindolinium; that is linked by a monomethine, trimethine, or pentamethine bridging moiety attached at the 2-position of said first ring system to the 2- or 4- position of a second heterocyclic ring system that is a substituted quinolinium, or that is linked by a monomethine bridging moiety attached to the 2-position of said first system to the 2- or 4-position of a second heterocyclic ring system that is a substituted pyridinium; wherein one or more substituents of said second ring system is an OMEGA, where OMEGA is a cyclohexyl, cyclohexenyl, morpholino, piperidinyl, naphthyl, phenyl, thienyl, benzothiazolyl, furanyl, oxazolyl, benzoxazolyl or pyridinyl, any of the afore said OMEGA groups being unsubstituted or optionally substituted one or more times, independently, by halogen, alkyl, perfluoroalkyl, amino, alkylamino, dialkylamino, alkoxy or carboxyalkyl, having 1-6 carbons, and, where there is more than one OMEGA, each OMEGA is the same or different.

7. A cyanine dye as claimed in claim 6, wherein
(i) said first heterocyclic ring system is substituted benzothiazolium or benzoxazolium, which first ring system is substituted at its aromatic nitrogen by a C₁-C₆ alkyl; and/or
(ii) said second ring system is further independently substituted by hydrogen, saturated or unsaturated C₁-C₆ alkyl, halogen, an ether, a thioether, a substituted or unsubstituted amino, an OMEGA; or a sulfonate ester.

8. A cyanine dye as claimed in claim 6 or 7, wherein said second ring system is a 4-quinolinium that is substituted at or adjacent to the ring nitrogen by an OMEGA.

9. A cyanine dye as claimed in any of claims 6 to 8, wherein OMEGA is a substituted or unsubstituted phenyl.

10. A cyanine dye as claimed in claim 8, wherein a substituent adjacent to OMEGA is hydrogen; a saturated or unsaturated alkyl; a halogen; an ether; a thioether; a substituted or unsubstituted amino, or a sulfonate ester.

11. A cyanine dye as claimed in claim 10, wherein said substituent adjacent to OMEGA is
(i) a halogen or a sulfonate ester; or
(ii) a thioether or substituted or unsubstituted amino.

12. A fluorescent complex comprising a nucleic acid and one or more molecules of a dye compound, which dye compound is either
A) of the formula: 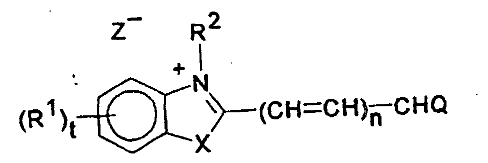 wherein
each R¹ is independently H; or C₁-C₆ alkyl; trifluoromethyl; a halogen; or -OR⁸, -SR⁸ or -(NR⁸R⁹) where R⁸ and R⁹, which can be the same or different, are independently H, C₁-C₆ alkyl or 1-2 alicyclic, heteroalicyclic, aromatic or heteroaromatic rings, having a total of 3-16 ring atoms, wherein the hetero atoms are O, N or S, or R⁸ and R⁹ taken in combination are -(CH₂)₂-L-(CH₂)₂- where L = a single bond, -O-, CH₂-, or -NR¹⁰-, where R¹⁰ is H or C₁-C₆ alkyl;
t = 1-4;
R² is C₁-C₆ alkyl;
X is O or S;
n = 0, 1 or 2;
Z⁻ is a biologically compatible counterion;
Q has the formula Q1 or Q2 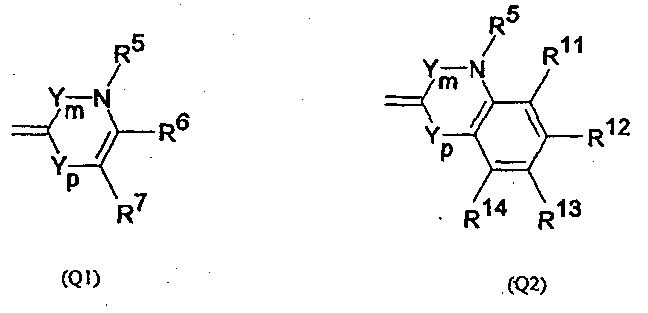
wherein
Y is -CR³=CR⁴-;
p and m = 0 or 1, such that p + m = 1;
R⁵ is an alkyl group having 1-6 carbons; or R⁵ is an OMEGA;
R³, R⁴, R⁶ and R⁷, which may be the same or different, are independently H; or an alkyl group having 1-6 carbons; or a halogen; or -OSO₂R¹⁹ where R¹⁹ is alkyl having 1-6 carbons, or perfluoroalkyl having 1-6 carbons, or aryl; or an OMEGA; or -OH, -OR⁸, -SR⁸, -(NR⁸R⁹);
or R⁶ and R⁷, taken in combination are -(CH₂)ᵥ₋ where v = 3 or 4, or R⁶ and R⁷ form a fused aromatic ring according to formula Q2;
R¹¹, R¹², R¹³, and R¹⁴, which may be the same or different, are independently H; or an alkyl group having 1-6 carbons; or a halogen; or an OMEGA; or -OH, -OR⁸, -SR⁸, or -(NR⁸R⁹);
OMEGA is a saturated or unsaturated, substituted or unsubstituted, cyclic substituent that has a total of 2-16 ring carbon atoms in 1-2 alicyclic, heteroalicyclic, aromatic, or heteroaromatic rings, containing 1-4 heteroatoms (wherein the hetero atoms are O, N or S), that is unsubstituted or optionally substituted one or more times, independently, by halogen, alkyl, perfluoroalkyl, amino, alkylamino, dialkylamino, alkoxy or carboxyalkyl, having 1-6 carbons, and that is attached as R³, R⁴, R⁵, R⁶, R⁷, R¹¹, R¹², R¹³ or R¹⁴ by a single bond, and where more than one of R³, R⁴, R⁵, R⁶, R⁷, R¹¹, R¹², R¹³ and R¹⁴ is an OMEGA, each OMEGA is the same or different;
provided that at least one of R³, R⁴, R⁵, R⁶, R⁷, R¹¹, R¹², R¹³ and R¹⁴ is an OMEGA; or is
B) a cyclic-substituted unsymmetrical cyanine dye, comprising a first heterocyclic ring system that is a substitued benzothiazolium, benzoxazolium, benzoselenazolium, benzimidazolium, or dialkylindolinium; that is linked by a monomethine, trimethine, or pentamethine bridging moiety attached at the 2-position of said first ring system to the 2- or 4-position of a second heterocyclic ring system that is a substituted pyridinium or quinolinium, wherein one or more substituents of said second ring system is an OMEGA, where OMEGA is a saturated or unsaturated, substituted or unsubstituted, cyclic substituent that has a total of 2-16 ring carbon atoms in 1-2 alicyclic, heteroalicyclic, aromatic, or heteroaromatic rings, containing 1-4 heteroatoms (wherein the heteroatoms are O, N or S).

13. A complex as claimed in claim 12, wherein the dye compound is as defined in any one of claims 1 to 5 or 6 to 11.

14. A method of staining nucleic acids in a sample, comprising
a) combining a sample that contains or is thought to contain nucleic acids with a cyclic substituted unsymmetrical dye compound as defined in claim 12 or claim 13 or in any of claims 1 to 11;
b) incubating the sample for a time sufficient for the dye compound to combine with the nucleic acids in the sample to form a nucleic acid-dye complex that gives a detectable fluorescent signal;
c) observing the detectable fluorescent signal of the nucleic acid-dye complex.

15. A method according to claim 14, wherein
(i) the nucleic acids are enclosed in a biological structure; or
(ii) the nucleic acids are enclosed in a biological structure and the method further comprises combining the sample with one or more additional dyes, singly or in combination; where at least one additional dye is a fluorescent nucleic acid stain that is permeant or impermeant to cells; another additional dye is selectively permeant to Gram positive or Gram negative bacteria; and where each of the additional dyes has a fluorescent response to illumination that is detectably different from that of the other dyes.

16. A method according to claim 15(ii) wherein
(i) the nucleic acid stain is impermeant to cells with intact membranes; or
(ii) the nucleic acid stain is impermeant to cells with intact membranes and is a phenanthridium monomer or dimer derivative that is an ethidium, ethidium dimer or propidium; or is a benzazolium monomer or dimer derivative.

17. A method according to claim 15(ii) wherein the nucleic acid stain is permeant to cells or is selectively permeant to Gram positive bacteria and is a C₄-C₈ alkyl-substituted phenanthridium, or 2'-(4-hydroxyphenyl)-5-(4-methyl-1-piperazinyl)-2,5'-bi-1H-benzimidazole, 2'-(4-ethoxyphenyl)-5-(4-methyl-1-piperazinyl)-2,5'-bi-1H-benzimidazole or 4', 6-diamidino-2-phenylindole dihydrochloride.

18. A method according to claim 14, wherein the nucleic acids are in solution.

19. A method according to claim 14 or 18 further comprising electrophoretic separation of the nucleic acids or nucleic acid-dye complex and optionally further comprising combining the dye compound with the gel before or after electrophoresis.

20. A method according to claim 14, of staining nucleic acids in a sample, wherein the nucleic acids are immobilized on a solid or semi-solid support.

21. A method according to any one of claims 14, 15(i), 15(ii), 16(i), 16(ii) or 17 further comprising
(i) combining the sample with one or more additional dyes, singly or in combination; where at least one additional dye is a fluorescent dye of a molecular weight of less than 2000 that selectively stains a cellular structure that is not a nucleic acid or is not an enzyme substrate; and where each of the additional dyes has a fluorescent response to illumination that is detectably different from that of the other dyes, wherein the cellular structure optionally is a cell membrane, a protein, a vacuole, a mitochondrion, a Golgi apparatus, an endoplasmic reticulum, a cytoplasm, a lysosome, or a saccharide or polysaccharide;
(ii) combining the sample with one or more additional dyes, singly or in combination; where at least one additional dye is a fluorescent peptide or protein, where the protein is an antibody, a lectin an avidin, streptavidin, protein A or protein G; and where each of the additional dyes has a fluorescent response to illumination that is detectably different from that of the other dyes; or
(iii) combining the sample with one or more additional dyes, singly or in combination; where at least one additional dye is a fluorogenic substrate for an intracellular enzyme; where said substrate has a fluorescent response to illumination that is detectably different from that of the other dyes after action of the enzyme, wherein the intracellular enzyme optionally is a hydrolytic enzyme, an oxidase or a reductase.

22. A method according to any one of claims 15(ii), 16(i), 16(ii) or 17 wherein the fluorescent response of the dyes to illumination is detected using a flow cytometer, a fluorometer or fluorescence plate reader, or a fluorescence microscope.

23. A compound of the formula: 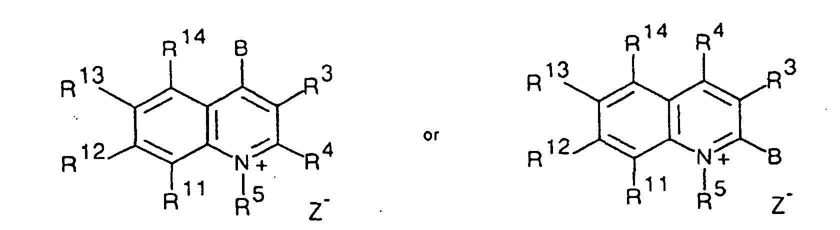 wherein R⁵ is an OMEGA as defined in claim 6 or claim 9;
B is methyl;
R³, R¹¹, R¹², R¹³, and R¹⁴ are independently H or alkyl having 1-6 carbons; and
R⁴ is F, Cl, Br, I, or -OSO₂R¹⁹ where R¹⁹ is alkyl having 1-6 carbons, or aryl.

24. A compound of the formula 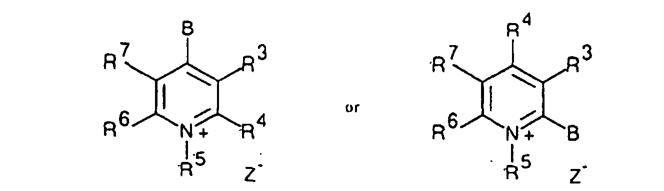 or of the formula 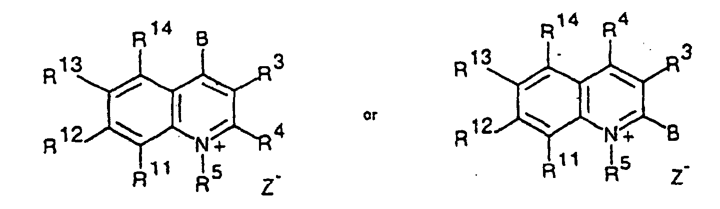 wherein R⁵ is an OMEGA as defined in claim 6 or claim 9;
B is methyl;
R⁶ and R⁷ are H;
R³, R¹¹, R¹², R¹³ and R¹⁴ are independently H or alkyl having 1-6 carbons; and
R⁴ is F, Cl, Br, I or -OSO₂R¹⁹ where R¹⁹ is alkyl having 1-6 carbons, or perfluoroalkyl having 1-6 carbons, or aryl.

25. A compound of claim 23 or claim 24 when substituted with a group forming at the 2-position an ether linkage, a thioether linkage or an amino group (whether substituted or unsubstituted).

26. A method of making a fluorescent dye, comprising conjoining a pyridinium or quinolinium compound as defined in claim 23 or claim 24 with a benzazolium compound of the formula 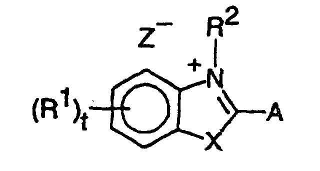 wherein R¹, t, X and Z are as defined in claim 1, and A is a substituent suitable for the conjoining of the compounds, to form a compound comprising a benzazolium ring system linked through its 2-position by 1, 3 or 5 bridging methine groups to the 4-position of a pyridinium or quinolinium ring system, and, when the pyridinium or quinolinium compound is as defined in claim 22 or claim 23, the method optionally further comprising condensing the pyridinium or quinolinium ring system at its 2-position with an alcohol, a phenol, an alkoxide, a thiol, a thiophenol, a thioether, ammonia or an amine.

27. A method of claim 26 wherein the resultant fluorescent dye has at the 2-position of the pyridinium or quinolinium ring system a substituent group forming an ether linkage, a thioether linkage or an amino group (whether substituted or unsubstituted).

28. A method of claim 27, wherein the group substituted at the 2-position of the pyridinium or quinolinium ring system comprises, in addition to any amino group directly bonded to the 2-position (the 2-position amino group), a substituted amino group, where the substituted amino group is in a heteroalicyclic ring which has from 3 to 16 ring atoms and which is bonded to the O, N or S atoms bonded to the 2-position of the ring system, and optionally wherein there is a 2-position amino group and the substituted amino group is group NR¹⁰ in a moiety of the formula -(CH₂)₂-NR¹⁰-(CH₂)₂- ring formingly bonded directly to the N atom of the 2-position amino group, R¹⁰ being H or 1C-6C alkyl.

29. The use to penetrate biological structures in detecting for nucleic acid of a compound as defined in claim 12 or claim 13 or in any of claims 1-11, wherein the biological structure optionally is:
(i) a viral particle,
(ii) an organelle, or
(iii) a cell.

30. The use of claim 29, wherein the pyridinium ring (group Q of claims 1 to 5) is of the formula 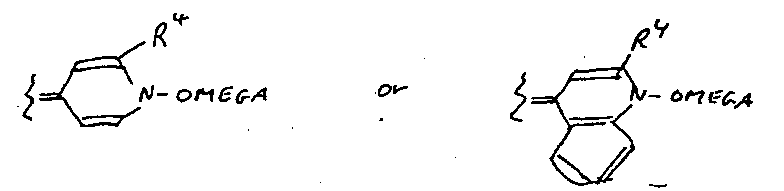 wherein
OMEGA is as defined in claim 1 or is a phenyl group and R⁴ is
(i) as defined in claim 1, or
(ii) selected from H, F, Cl, Br, n-butyl, -NH-phenyl, NEt₂, N-nPr₂, N-methylpiperazyl, morpholinyl, anilinyl, 2-(N-3-dimethylaminopropyl)-N-propylamino, Me, OMe, OEt, OPh, Ph, SMe, O-iPr, cyclohexyl, S-2-pyridyl, 2-(2-dimethylaminoethylthio) and OSO₂CF₃.

## Patentansprüche

1. Verbindung der Formel 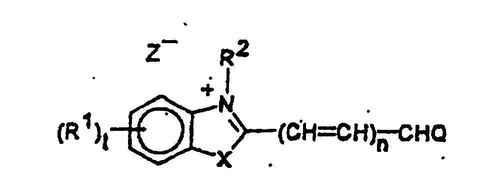 worin
R¹ jeweils unabhängig für H; C₁-C₆-Alkyl; Trifluormethyl; ein Halogen; oder -OR⁸, -SR⁸ oder -(NR⁸R⁹), wobei R⁸ und R⁹, welche gleich oder verschieden sein können, unabhängig H, C₁-C₆-Alkyl oder 1-2 alicyclische, heteroalicyclische, aromatische oder heteroaromatische Ringe mit insgesamt 3-16 Ringatomen sind, worin die Heteroatome O, N oder S sind oder R⁸ und R⁹ zusammengenommen - (CH₂)₂-L- (CH₂)₂ bedeuten, wobei L=Einzelbindung, -O-, CH₂- oder -NR¹⁰-, wobei R¹⁰ H oder C₁-C₆-Alkyl ist, steht;
t=1-4;
R² für C₁-C₆-Alkyl steht;
X für O oder S steht;
n=0, 1 oder 2;
Z für ein biologisch verträgliches Gegenion steht;
Q die Formel Q1 oder Q2 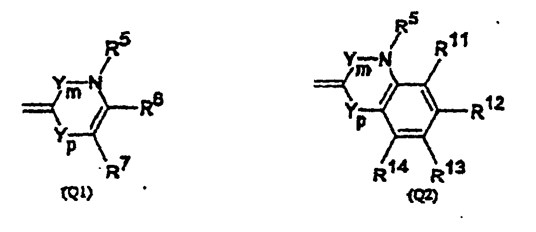 aufweist, worin
Y für -CR³=CR⁴- steht;
p und m=0 oder 1, so daß p+m=1;
R⁵ für C₁-C₆-Alkyl oder ein OMEGA steht;
R³, R⁴, R⁶ und R⁷, welche gleich oder verschieden sein können, unabhängig für H, C₁-C₆-Alkyl, ein Halogen oder -OSO₂R¹⁹, wobei R¹⁹ Alkyl mit 1-6 C-Atomen oder Perfluoralkyl mit 1-6 C-Atomen oder Aryl ist, oder ein OMEGA, oder -OH, -OR⁸, -SR⁸, -(NR⁸R⁹) stehen;
oder R⁶ und R⁷ zusammengenommen für -(CH₂)ᵥ-stehen, wobei v=3 oder 4, oder R⁶ und R⁷ einen kondensierten aromatischen Ring gemäß Formel Q2 bilden;
R¹¹, R¹², R¹³ und R¹⁴, welche gleich oder verschieden sein können, unabhängig für H oder eine Alkylgruppe mit 1-6 C-Atomen oder ein Halogen oder ein OMEGA oder -OH, -OR⁸, -SR⁸ oder -(NR⁸R⁹) stehen;
OMEGA für Cyclohexyl, Cyclohexenyl, Morpholino, Piperidinyl, Naphthyl, Phenyl, Thienyl, Benzothiazolyl, Furanyl, Oxazolyl, Benzoxazolyl oder Pyridinyl, wobei einer der vorgenannten OMEGA-Gruppen unsubstituiert oder gegebenenfalls ein- oder mehrfach unabhängig mit Halogen, Alkyl, Perfluoralkyl, Amino, Alkylamino, Dialkylamino, Alkoxy oder Carboxyalkyl mit 1-6 C-Atomen substituiert ist und über eine Einzelbindung als R³, R⁴, R⁵, R⁶, R⁷, R¹¹, R¹², R¹³ oder R¹⁴ gebunden ist, und, falls mehr als einer der Reste R³, R⁴, R⁵, R⁶, R⁷, R¹¹, R¹², R¹³ oder R¹⁴ ein OMEGA ist, jedes OMEGA gleich oder verschieden ist, steht;
mit der Maßgabe, daß wenigstens einer der Reste R³, R⁴, R⁵, R⁶, R⁷, R¹¹, R¹², R¹³ und R¹⁴ ein OMEGA ist und daß, wenn Q die Formel Q1 aufweist, n=0.

2. Verbindung gemäß Anspruch 1, wobei m=1 und R⁵ für OMEGA steht.

3. Verbindung gemäß Anspruch 1 oder 2, wobei R⁴ für H steht; oder R⁴ für eine Alkylgruppe mit 1-6 C-Atomen oder ein Halogen steht; oder R⁴ für -OH, -OR⁸, -SR⁸, - (NR⁸R⁹) steht; oder R⁴ für OSO₂R¹⁹ steht; oder R⁴ für ein OMEGA steht.

4. Verbindung gemäß Anspruch 3, wobei n=0 oder 1 und OMEGA für Phenyl oder substituiertes Phenyl steht.

5. Verbindung gemäß Anspruch 3 oder 4, wobei
(i) R⁴ für Halogen oder -OSO₂R¹⁹ steht; oder
(ii) R⁴ für -SR⁸ oder - (NR⁸R⁹) steht.

6. Cyclisch substituierter unsymmetrischer Cyaninfarbstoff, enthaltend ein erstes heterocyclisches Ringsystem, welches ein substituiertes Benzothiazolium, Benzoxazolium, Benzoselenazolium, Benzimidazolium oder Dialkylindolinium; welches über eind an der 2-Position des ersten Ringsystems befindliche Monomethin-, Trimethin- oder Pentamethin-Brücke mit der 2- oder 4-Position eines zweiten heterocyclischen Ringsystems, welches ein substituiertes Chinolinium ist, verknüpft ist, oder welches über eine an der 2-Position des ersten Systems befindliche Monomethin-Brücke mit der 2- oder 4-Position eines zweiten heterocyclischen Ringsystems, welches ein substituiertes Pyridinium ist, verknüpft ist; worin ein oder mehrere Substituenten des zweiten Ringsystems ein OMEGA ist, wobei OMEGA ein Cyclohexyl, Cyclohexenyl, Morpholino, Piperidinyl, Naphthyl, Phenyl, Thienyl, Benzothiazolyl, Furanyl, Oxazolyl, Benzoxazolyl oder Pyridinyl ist, wobei eine der vorgenannten OMEGA-Gruppen unsubstituiert oder gegebenenfalls ein- oder mehrfach unabhängig mit Halogen, Alkyl, Perfluoralkyl, Amino, Alkylamino, Dialkylamino, Alkoxy oder Carboxyalkyl mit 1-6 C-Atomen substituiert ist, und, falls mehr als ein OMEGA vorhanden ist, jedes OMEGA gleich oder verschieden ist, ist.

7. Cyaninfarbstoff gemäß Anspruch 6, wobei
(i) das an seinem aromatischen Stickstoff mit einem C₁-C₆-Alkyl substituierte erste heterocyclische Ringsystem ein substituiertes Benzothiazolium oder Benzoxazolium ist; und/oder
(ii) das zweite Ringsystem weiterhin unabhängig mit Wasserstoff, gesättigtem oder ungesättigtem C₁-C₆-Alkyl, Halogen, einem Ether, einem Thioether, einem gegebenenfalls substituierten Amino, einem OMEGA oder einem Sulfonsäureester substituiert ist.

8. Cyaninfarbstoff gemäß Anspruch 6 oder 7, wobei das zweite Ringsystem ein 4-Chinolinium ist, welches an oder neben dem Ringstickstoff mit einem OMEGA substituiert ist.

9. Cyaninfarbstoff gemäß einem der Ansprüche 6 bis 8, wobei OMEGA ein gegebenenfalls substituiertes Phenyl ist.

10. Cyaninfarbstoff gemäß Anspruch 8, wobei ein zu OMEGA benachbarter Substituent, Wasserstoff, ein gesättigtes oder ungesättigtes Alkyl, ein Halogen, ein Ether, ein Thioether, ein gegebenenfalls substituiertes Amino oder ein Sulfonsäureester ist.

11. Cyaninfarbstoff gemäß Anspruch 10, wobei der zu OMEGA benachbarte Substituent
(i) ein Halogen oder ein Sulfonsäureester, oder
(ii) ein Thioether oder ein gegebenenfalls substituiertes Amino ist.

12. Fluoreszierender Komplex, enthaltend eine Nukleinsäure und ein oder mehrere Moleküle einer Farbstoffverbindung, welche entweder
A) die Formel 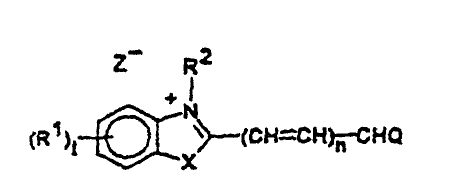 aufweist, worin
R¹ jeweils unabhängig für H; oder C₁-C₆-Alkyl; Trifluormethyl; ein Halogen; oder -OR⁸, -SR⁸ oder -(NR⁸R⁹), wobei R⁸ und R⁹, welche gleich oder verschieden sein können, unabhängig H, C₁-C₆-Alkyl oder 1-2 alicyclische, heteroalicyclische, aromatische oder heteroaromatische Ringe mit insgesamt 3-16 Ringatomen sind, worin die Heteroatome O, N oder S sind oder R⁸ und R⁹ zusammengenommen -(CH₂)₂-L- (CH₂)₂ bedeuten, wobei L=Einzelbindung, -O-, CH₂- oder -NR¹⁰-, wobei R¹⁰ H oder C₁-C₆-Alkyl ist, steht;
t=1-4;
R² für C₁-C₆-Alkyl steht;
X für O oder S steht;
n=0, 1 oder 2;
Z⁻ für ein biologisch verträgliches Gegenion steht;
Q die Formel Q1 oder Q2 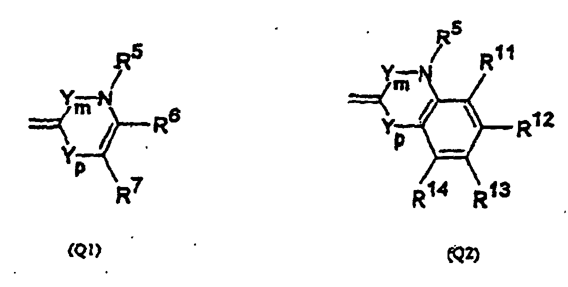 aufweist, worin
Y für -CR³=CR⁴- steht;
p und m=0 oder 1, so daß p+m=1;
R⁵ für eine Alkylgruppe mit 1-6 C-Atomen steht; oder R⁵ für ein OMEGA steht;
R³, R⁴, R⁶ und R⁷, welche gleich oder verschieden sein können, unabhängig für H, eine Alkylgruppe mit 1-6 C-Atomen, oder ein Halogen oder -OSO₂R¹⁹, wobei R¹⁹ Alkyl mit 1-6 C-Atomen oder Perfluoralkyl mit 1-6 C-Atomen oder Aryl ist, oder ein OMEGA, oder -OH, -OR⁸, -SR⁸, - (NR⁸R⁹) stehen;
oder R⁶ und R⁷ zusammengenommen für -(CH₂)ᵥ-stehen, wobei v=3 oder 4, oder R⁶ und R⁷ einen kondensierten aromatischen Ring gemäß Formel Q2 bilden;
R¹¹, R¹², R¹³ und R¹⁴, welche gleich oder verschieden sein können, unabhängig für H oder eine Alkylgruppe mit 1-6 C-Atomen oder ein Halogen oder ein OMEGA oder -OH, -OR⁸, -SR⁸ oder -(NR⁸R⁹) stehen;
OMEGA für einen gesättigten oder ungesättigten gegebenenfalls substituierten cyclischen Substituenten mit insgesamt 2-16 Ringkohlenstoffatomen in 1-2 alicyclischen, heteroalicyclischen, aromatischen oder heteroaromatischen, 1-4 Heteroatome (worin die Heteroatome O, N oder S sind) enthaltenden Ringen, welcher unsubstituiert oder gegebenenfalls ein- oder mehrfach unabhängig mit Halogen, Alkyl, Perfluoralkyl, Amino, Alkylamino, Dialkylamino, Alkoxy oder Carboxyalkyl mit 1-6 C-Atomen substituiert ist und über eine Einzelbindung als R³, R⁴, R⁵, R⁶, R⁷, R¹¹, R¹², R¹³ oder R¹⁴ gebunden ist, und, falls mehr als einer der Reste R³, R⁴, R⁵, R⁶, R⁷, R¹¹, R¹², R¹³ und R¹⁴ ein OMEGA ist, jedes OMEGA gleich oder verschieden ist, steht;
mit der Maßgabe, daß wenigstens einer der Reste R³, R⁴, R⁵, R⁶, R⁷, R¹¹, R¹², R¹³ und R¹⁴ ein OMEGA ist; oder
B) cyclisch substituierter unsymmetrischer Cyaninfarbstoff, enthaltend ein erstes heterocyclisches Ringsystem, welches ein substituiertes Benzothiazolium, Benzoxazolium, Benzoselenazolium, Benzimidazolium oder Dialkylindolinium; welches über eine an der 2-Position des ersten Ringsystems befindliche Monomethin-, Trimethin- oder Pentamethin-Brücke mit der 2- oder 4-Position eines zweiten heterocyclischen Ringsystems, welches ein substituiertes Pyridinium oder Chinolinium ist, verknüpft ist, worin ein oder mehrere Substituenten des zweiten Ringsystems ein OMEGA ist, wobei OMEGA ein gesättigter oder ungesättigter gegebenenfalls substituierter cyclischer Substituent mit insgesamt 2-16 Ringkohlenstoffatomen in 1-2 alicyclischen, heteroalicyclischen, aromatischen oder heteroaromatischen, 1-4 Heteroatome (worin die Heteroatome O, N oder S sind) enthaltenden Ringen ist, ist.

13. Komplex gemäß Anspruch 12, wobei die Farbstoffverbindung wie unter einem der Ansprüche 1 bis 5 oder 6 bis 11 definiert ist.

14. Verfahren zum Anfärben von Nukleinsäuren in einer Probe, bei dem man
a) eine Probe, die Nukleinsäuren enthält oder enthalten könnte, mit einer cyclisch substituierten unsymmetrischen Farbstoffverbindung wie in Anspruch 12 oder in Anspruch 13 oder in einem der Ansprüche 1 bis 11 definiert kombiniert;
b) die Probe solange inkubiert, daß man die Farbstoffverbindung mit den Nukleinsäuren in der Probe kombinieren kann, um einen Nukleinsäure-Farbstoffkomplex zu bilden, der ein nachweisbares Fluoreszenzsignal liefert;
c) das nachweisbare Fluoreszenzsignal des Nukleinsäure-Farbstoffkomplexes beobachtet.

15. Verfahren gemäß Anspruch 14, wobei
(i) die Nukleinsäuren von einer biologischen Struktur umschlossen sind; oder
(ii) die Nukleinsäuren von einer biologischen Struktur umschlossen sind und das Verfahren weiterhin umfaßt, daß man die Probe mit einem oder mehreren zusätzlichen Farbstoffen einzeln oder zusammen kombiniert; wobei wenigstens ein zusätzlicher Farbstoff ein fluoreszierender Nukleinsäurefarbstoff ist, der gegebenenfalls in Zellen eindringen kann; ein weiterer zusätzlicher Farbstoff gram-positive oder gram-negative Bakterien selektiv durchdringen kann; und wobei jeder der zusätzlichen Farbstoffe mit einem zu dem der anderen Farbstoffe nachweisbar verschiedenen Fluoreszenzsignal auf Belichtung reagiert.

16. Verfahren gemäß Anspruch 15 (ii), wobei
(i) der Nukleinsäurefarbstoff nicht in Zellen mit intakten Membranen eindringen kann; oder
(ii) der Nukleinsäurefarbstoff nicht in Zellen mit intakten Membranen eindringen kann und ein Phenanthridiummonomer- oder -dimerderivat ist, welches ein Ethidium, Ethidiumdimer oder Propidium ist; oder ein Benzazoliummonomer- oder - dimerderivat ist.

17. Verfahren gemäß Anspruch 15(ii), wobei der Nukleinsäurefarbstoff in Zellen oder selektiv in gram-positive Bakterien eindringen kann und ein C₄-C₈-alkylsubstituiertes Phenanthridium oder 2'-(4-Hydroxyphenyl)-5-(4-methyl-1-piperazinyl)-2,5'-bi-1H-benzimidazol, 2'-(4-Ethoxyphenyl)-5-(4-methyl-1-piperazinyl)-2,5'-bi-1H-benzimidazol oder 4',6-Diamidino-2-phenylindoldihydrochlorid ist.

18. Verfahren gemäß Anspruch 14, wobei die Nukleinsäuren in Lösung vorliegen.

19. Verfahren gemäß Anspruch 14 oder 18, weiterhin umfassend elektrophoretische Trennung der Nukleinsäuren oder des Nukleinsäure-Farbstoffkomplexes und gegebenenfalls weiterhin umfassend, daß man die Farbstoffverbindung mit dem Gel vor oder nach der Elektrophorese kombiniert.

20. Verfahren gemäß Anspruch 14 zum Anfärben von Nukleinsäuren in einer Probe, wobei die Nukleinsäuren auf einem festen oder halbfesten Träger immobilisiert sind.

21. Verfahren gemäß einem der Ansprüche 14, 15(i), 15(ii), 16(i), 16(ii) oder 17, weiterhin umfassend, daß man
(i) die Probe mit einem oder mehreren zusätzlichen Farbstoffen einzeln oder zusammen kombiniert; wobei wenigstens ein zusätzlicher Farbstoff ein Fluoreszenzfarbstoff mit einem Molekulargewicht von weniger als 2000 ist, welcher selektiv eine Zellstruktur anfärbt, die weder eine Nukleinsäure noch ein Enzymsubstrat ist; und wobei jeder der zusätzlichen Farbstoffe mit einem von dem der anderen Farbstoffe nachweisbar verschiedenen Fluoreszenzsignal auf Belichtung reagiert, wobei die Zellstruktur gegebenenfalls eine Zellmembran, ein Protein, eine Vacuole, ein Mitochondrion, ein Golgi-Apparat, ein endoplasmatisches Reticulum, ein Cytoplasma, ein Lysosom oder ein Saccharid oder Polysaccharid ist;
(ii) die Proben mit einem oder mehreren zusätzlichen Farbstoffen einzeln oder zusammen kombiniert; wobei wenigstens ein zusätzlicher Farbstoff ein fluoreszierendes Peptid oder Protein ist, wobei das Protein ein Antikörper, ein Lectin, ein Avidin, Streptavidin, Protein A oder Protein G ist; und wobei jeder der zusätzlichen Farbstoffe mit einem von dem der anderen Farbstoffe nachweisbar verschiedenen Fluoreszenzsignal auf Belichtung reagiert; oder
(iii) die Probe mit einem oder mehreren zusätzlichen Farbstoffen einzeln oder zusammen kombiniert; wobei wenigstens ein zusätzlicher Farbstoff ein fluorogenes Substrat für ein intrazelluläres Enzym ist; wobei nach der Enzymreaktion das Substrat mit einem von dem der anderen Farbstoffe nachweisbar verschiedenen Fluoreszenzsignal auf Belichtung reagiert, wobei das intrazelluläre Enzym gegebenenfalls ein hydrolytisches Enzym, eine Oxidase oder eine Reduktase ist.

22. Verfahren gemäß einem der Ansprüche 15(ii), 16(i), 16(ii) oder 17, wobei die Fluoreszenzreaktion der Farbstoffe auf Belichtung mit einem Durchflußzytometer, einem Fluoreszenzmeßgerät oder Fluoreszenz-Plattenlesegerät oder einem Fluoreszenzmikroskop nachgewiesen wird.

23. Verbindung der Formel: 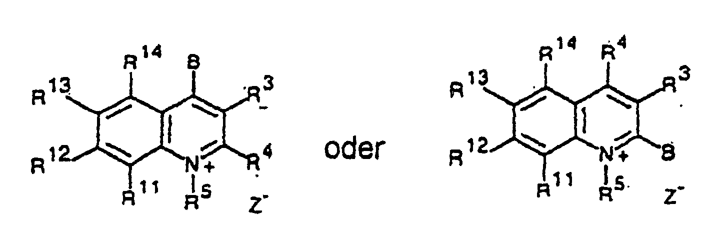 worin R⁵ für ein OMEGA wie in Anspruch 6 oder Anspruch 9 definiert steht;
B für Methyl steht;
R³, R¹¹, R¹², R¹³ und R¹⁴ unabhängig für H oder Alkyl mit 1-6 C-Atomen stehen; und
R⁴ für F, Cl, Br, J oder -OSO₂R¹⁹, wobei R¹⁹ für Alkyl mit 1-6 C-Atomen steht, oder Aryl steht.

24. Verbindung der Formel 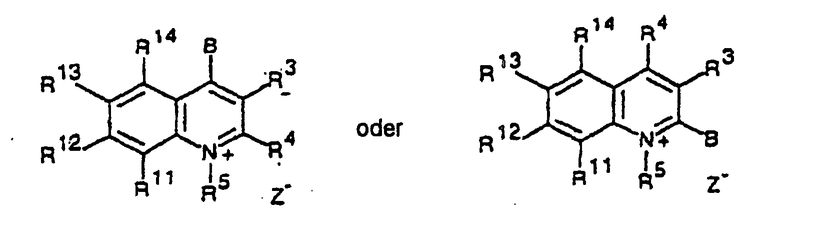 oder der Formel 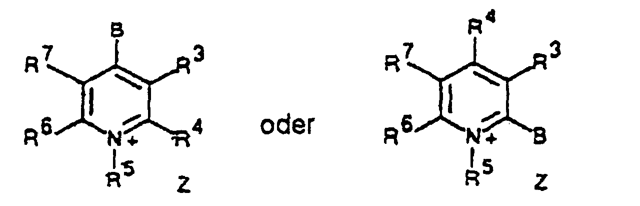 worin R⁵ für ein OMEGA wie in Anspruch 6 oder Anspruch 9 definiert steht;
B für Methyl steht;
R⁶ und R⁷ für H stehen;
R³, R¹¹, R¹², R¹³ und R¹⁴ unabhängig für H oder Alkyl mit 1-6 C-Atomen stehen; und
R⁴ für F, Cl, Br, J oder -OSO₂R¹⁹, wobei R¹⁹ für Alkyl mit 1-6 C-Atomen steht, oder Perfluoralkyl mit 1-6 C-Atomen oder Aryl steht.

25. Verbindung nach Anspruch 23 oder 24, wobei die Verbindung mit einer Gruppe, die in 2-Position eine Etherverknüpfung, eine Thioetherverknüpfung oder eine (gegebenenfalls substituierte) Aminogruppe bildet, substituiert ist.

26. Verfahren zur Herstellung eines Fluoreszenzfarbstoffes, bei dem man eine Pyridinium- oder Chinoliniumverbindung wie in Anspruch 23 oder Anspruch 24 definiert mit einer Benzazoliumverbindung der Formel 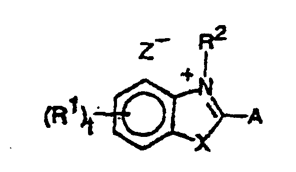 verbindet, worin R¹, t, X und Z wie in Anspruch 1 definiert sind und A ein für die Verknüpfung der Verbindungen geeigneter Substituent ist, um eine Verbindung zu bilden, die aus einem Benzazoliumringsystem besteht, das durch seine 2-Position über 1, 3 oder 5 Methinbrücken mit der 4-Position eines Pyridinium- oder Chinolinium-Ringsystems verknüpft ist, wobei, wenn die Pyridinium- oder Chinoliniumverbindung wie in Anspruch 22 oder Anspruch 23 definiert ist, das Verfahren gegebenenfalls weiterhin umfaßt, daß man das Pyridinium- oder Chinolinium-Ringsystem an seiner 2-Position mit einem Alkohol, einem Phenol, einem Alkoholat, einem Thiol, einem Thiophenol, einem Thioether, Ammoniak oder einem Amin kondensiert.

27. Verfahren gemäß Anspruch 26, wobei der entstandene Fluoreszenzfarbstoff eine Substituentengruppe, die eine Etherverknüpfung, eine Thioetherverknüpfung oder eine (gegebenenfalls substituierte) Aminogruppe bildet, in der 2-Position des Pyridinium- oder Chinolinium-Ringsystems aufweist.

28. Verfahren gemäß Anspruch 27, wobei die in der 2-Position des Pyridinium- oder Chinolinium-Ringsystems substituierte Gruppe neben einer beliebigen direkt an der 2-Position gebundenen Aminogruppe (die 2-Position-Aminogruppe) eine substituierte Aminogruppe umfaßt, wobei die substituierte Aminogruppe in einem heteroalicyclischen Ring mit von 3 bis 16 Ringatomen vorliegt, der an die an die 2-Position des Ringsystems gebundenen O-, N- oder S-Atome gebunden ist, und wobei gegebenenfalls eine 2-Position-Aminogruppe vorliegt und die substituierte Aminogruppe die Gruppe NR¹⁰ in einem Teil der Formel -(CH₂)₂-NR¹⁰-(CH₂)₂- ist, die unter Ringbildung direkt an das N-Atom der 2-Position-Aminogruppe gebunden ist, wobei R¹⁰ für H oder C₁-C₆-Alkyl steht.

29. Verwendung einer Verbindung wie in Anspruch 12 oder Anspruch 13 oder in einem der Ansprüche 1-11 definiert zum Durchdringen biologischer Strukturen für den Nachweis von Nukleinsäuren, wobei die biologische Struktur gegebenenfalls:
(i) ein Viruspartikel,
(ii) eine Organelle oder
(iii) eine Zelle ist.

30. Verwendung gemäß Anspruch 29, wobei der Pyridiniumring (Gruppe Q der Ansprüche 1 bis 5) die Formel 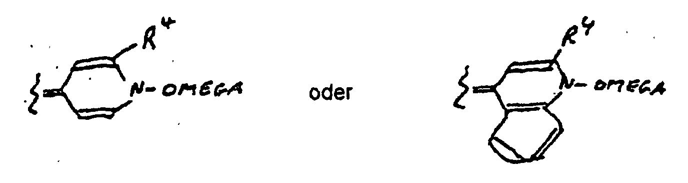 aufweist, worin
OMEGA wie in Anspruch 1 definiert ist oder für eine Phenylgruppe steht und R⁴
(i) wie in Anspruch 1 definiert ist oder
(ii) ausgewählt ist aus H, F, Cl, Br, n-Butyl, -NH-Phenyl, NEt₂, N-nPr₂, N-Methylpiperazyl, Morpholinyl, Anilinyl, 2-(N-3-Dimethylaminopropyl)-N-propylamino, Me, OMe, OEt, OPh, Ph, SMe, O-iPr, Cyclohexyl, S-2-Pyridyl, 2-(2-Dimethylaminoethylthio) und OSO₂CF₃.

## Revendications

1. Composé de formule 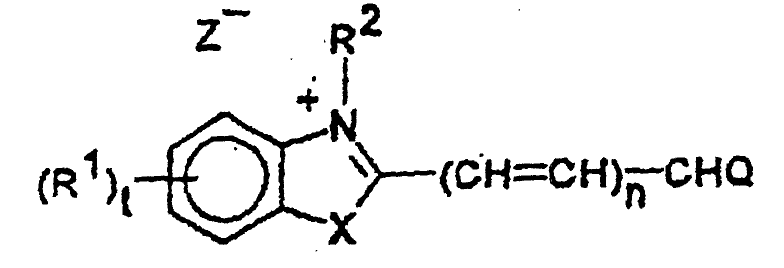 dans laquelle
chaque R¹ est indépendamment H ; alkyle en C₁-C₆ ; trifluorométhyle ; un halogène ; ou -OR⁸, -SR⁸ ou -(NR⁸R⁹) où R⁸ et R⁹, qui peuvent être identiques ou différents, sont indépendamment H, alkyle en C₁-C₆ ou 1-2 cycles alicycliques, hétéroalicycliques, aromatiques ou hétéroaromatiques, ayant un total de 3-16 atomes cycliques, dans lequel les hétéroatomes sont O, N ou S, ou R⁸ et R⁹ pris en combinaison sont -(CH₂)₂-L-(CH₂)₂- où L = une liaison simple, -O-, -CH₂-, -NR¹⁰ où R¹⁰ est H ou alkyle en C₁-C₆ ;
t=1-4 ;
R² est alkyle en C₁-C₆ ;
X est O ou S ;
n=0, 1 ou 2 ;
Z est un contre-ion biologiquement compatible ;
Q a la formule Q1 ou Q2 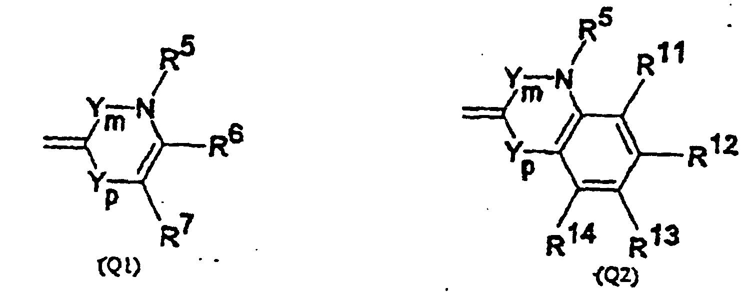
dans laquelle
Y est -CR³=CR⁴- ;
p et m = 0 ou 1, tels que p+m=1 ;
R⁵ est alkyle en C₁-C₆ ou un OMEGA ;
R³, R⁴, R⁶ et R⁷, qui peuvent être identiques ou différents, sont indépendamment H, alkyle en C₁-C₆, un halogène, ou -OSO₂R¹⁹ où R¹⁹ est alkyle ayant 1-6 carbones, ou perfluoroalkyle ayant 1-6 carbones, ou aryle ; ou un OMEGA ; ou -OH, -OR⁸, -SR⁸ ou -(NR⁸R⁹) ;
ou R⁶ et R⁷, pris en combinaison sont -(CH₂)ᵥ-, où v=3 ou 4, ou R⁶ et R⁷ forment un cycle aromatique fusionné selon la formule Q2 ;
R¹¹, R¹², R¹³ et R¹⁴, qui peuvent être identiques ou différents, sont indépendamment H ; ou un groupement alkyle ayant 1-6 carbones ; ou un halogène ; ou un OMEGA ; ou -OH, -OR⁸, -SR⁸ ou -(NR⁸R⁹) ;
OMEGA est cyclohexyle, cylohexényle, morpholino, pipéridinyle, naphtyle, phényle, thiényle, benzothiazolyle, furannyle, oxazolyle, benzoxazolyle ou pyridinyle, n'importe lequel des groupements OMEGA ci-avant étant non substitué ou facultativement substitué une ou plusieurs fois, indépendamment, par halogène, alkyle, perfluoroalkyle, amino, alkylamino, dialkylamino, alkoxy ou carboxyalkyle, ayant 1-6 carbones, et qui est attaché comme R³, R⁴, R⁵, R⁶, R⁷, R¹¹, R¹², R¹³ ou R¹⁴ par une liaison simple, et, où plus d'un des R³, R⁴, R⁵, R⁶, R⁷, R¹¹, R¹², R¹³ ou R¹⁴ est un OMEGA, chaque OMEGA est identique ou différent ;
à condition qu'au moins un des R³, R⁴, R⁵, R⁶, R⁷, R¹¹, R¹², R¹³ et R¹⁴ soit un OMEGA et que quand Q a la formule Q1, n=0.

2. Composé selon la revendication 1 dans lequel m=1 et R⁵ est un OMEGA.

3. Composé selon la revendication 1 ou la revendication 2 dans lequel R⁴ est H ; ou R⁴ est un groupement alkyle ayant 1-6 carbones ; ou un halogène ; ou R⁴ est -OH, -OR⁸, -SR⁸ ou -(NR⁸R⁹) ; ou R⁴ est -OSO₂R¹⁹ ; ou R⁴ est un OMEGA.

4. Composé selon la revendication 3 dans lequel n=0 ou 1, et OMEGA est un phényle ou un phényle substitué.

5. Composé selon la revendication 3 ou la revendication 4, dans lequel
(i) R⁴ est halogène ou -OSO₂R¹⁹ ; ou
(ii) R⁴ est -SR⁸ ou -(NR⁸R⁹).

6. Colorant de cyanine asymétrique substitué de manière cyclique, comprenant un premier système hétérocyclique qui est un benzothiazolium, benzoxazolium, benzosélénazolium, benzimidazolium ou dialkylindolinium substitué ; qui est lié par une partie de pont monométhine, triméthine ou pentaméthine attachée en position 2 dudit premier système cyclique en position 2 ou 4 d'un second système hétérocyclique qui est un quinolinium substitué, ou qui est lié par une partie de pont monométhine attachée en position 2 dudit premier système en position 2 ou 4 d'un second système hétérocyclique qui est un pyridinium substitué ; dans lequel un ou plusieurs substituants dudit second système cyclique est un OMEGA, où OMEGA est un cyclohexyle, cylohexényle, morpholino, pipéridinyle, naphtyle, phényle, thiényle, benzothiazolyle, furannyle, oxazolyle, benzoxazolyle ou pyridinyle, n'importe lequel des groupements OMEGA ci-avant étant non substitué ou facultativement substitué une ou plusieurs fois, indépendamment, par halogène, alkyle, perfluoroalkyle, amino, alkylamino, dialkylamino, alkoxy ou carboxyalkyle, ayant 1-6 carbones, et, où il y a plus d'un OMEGA, chaque OMEGA est identique ou différent.

7. Colorant de cyanine selon la revendication 6 dans lequel
(i) ledit premier système hétérocyclique est un benzothiazolium ou benzoxazolium substitué, lequel premier système cyclique est substitué à son azote aromatique par un alkyle en C₁-C₆ ; et/ou
(ii) ledit second système cyclique est en outre indépendamment substitué par hydrogène, alkyle en C₁-C₆ saturé ou non saturé, halogène, un éther, un thioéther, un amino substitué ou non substitué, un OMEGA ; ou un ester sulfonate.

8. Colorant de cyanine selon la revendication 6 ou 7, dans lequel ledit second système cyclique est un 4-quinolinium qui est substitué à ou adjacent à l'azote cyclique par un OMEGA.

9. Colorant de cyanine selon l'une quelconque des revendications 6 à 8, dans lequel OMEGA est un phényle substitué ou non substitué.

10. Colorant de cyanine selon la revendication 8, dans lequel un substituant adjacent à OMEGA est hydrogène ; un alkyle saturé ou insaturé ; un halogène ; un éther ; un thioéther ; un amino substitué ou non substitué ; ou un ester sulfonate.

11. Colorant de cyanine selon la revendication 10, dans lequel ledit substituant adjacent à OMEGA est
(i) un halogène ou un ester sulfonate ; ou
(ii) un thioéther ou un amino substitué ou non substitué.

12. Complexe fluorescent comprenant un acide nucléique et une ou plusieurs molécules d'un composé de colorant, composé de colorant qui est soit
A) de formule 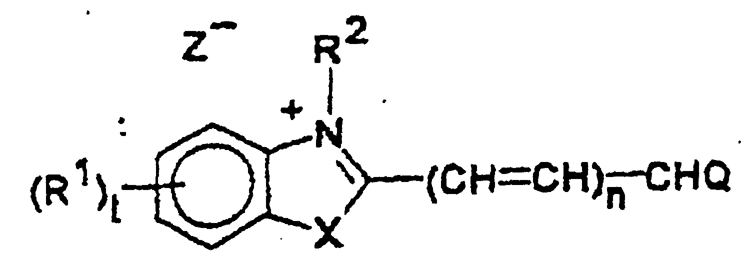 dans laquelle
chaque R¹ est indépendamment H ; ou alkyle en C₁-C₆ ; trifluorométhyle ; un halogène ; ou -OR⁸, -SR⁸ ou -(NR⁸R⁹) où R⁸ et R⁹, qui peuvent être identiques ou différents, sont indépendamment H, alkyle en C₁-C₆ ou 1-2 cycles alicycliques, hétéroalicycliques, aromatiques ou hétéroaromatiques, ayant un total de 3-16 atomes cycliques, dans lequel les hétéroatomes sont O, N ou S, ou R⁸ et R⁹ pris en combinaison sont -(CH₂)₂-L-(CH₂)₂- où L = une liaison simple, -O-, -CH₂- ou -NR¹⁰ où R¹⁰ est H ou alkyle en C₁-C₆ ;
t=1-4 ;
R² est alkyle en C₁-C₆ ;
X est O ou S ;
n=0, 1 ou 2 ;
Z est un contre-ion biologiquement compatible ;
Q a la formule Q1 ou Q2 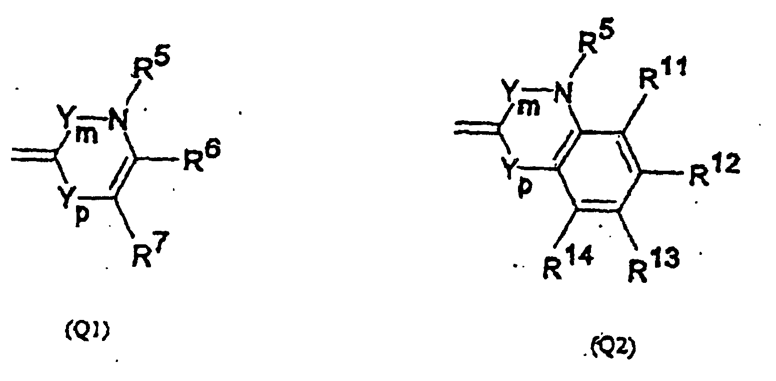 dans laquelle
Y est -CR³=CR⁴- ;
p et m = 0 ou 1, tels que p+m=1 ;
R⁵ est un groupement alkyle ayant 1-6 carbones ; ou R⁵ est un OMEGA ;
R³, R⁴, R⁶ et R⁷, qui peuvent être identiques ou différents, sont indépendamment H ; ou un groupement alkyle ayant 1-6 carbones ; ou un halogène ; ou -OSO₂R¹⁹ où R¹⁹ est alkyle ayant 1-6 carbones, ou perfluoroalkyle ayant 1-6 carbones, ou aryle ; ou un OMEGA ; ou -OH, -OR⁸, -SR⁸ ou - (NR⁸R⁹) ;
ou R⁶ et R⁷, pris en combinaison sont - (CH₂)ᵥ-, où v=3 ou 4, ou R⁶ et R⁷ forment un cycle aromatique fusionné selon la formule Q2 ;
R¹¹, R¹², R¹³ et R¹⁴, qui peuvent être identiques ou différents, sont indépendamment H ; ou un groupement alkyle ayant 1-6 carbones ; ou un halogène ; ou un OMEGA ; ou -OH, -OR⁸, -SR⁸ ou -(NR⁸R⁹) ;
OMEGA est un substituant cyclique saturé ou insaturé, substitué ou non substitué, qui a un total de 2-16 atomes de carbone cyclique dans 1-2 cycles alicyliques, hétéroalicycliques, aromatiques ou hétéroaromatiques contenant 1-4 hétéroatomes (dans lequel les hétéroatomes sont O, N ou S), qui est non substitué ou facultativement substitué une ou plusieurs fois, indépendamment, par halogène, alkyle, perfluoroalkyle, amino, alkylamino, dialkylamino, alkoxy ou carboxyalkyle, ayant 1-6 carbones, et qui est attaché comme R³, R⁴, R⁵, R⁶, R⁷, R¹¹, R¹², R¹³ ou R¹⁴ par une liaison simple, et, où plus d'un des R³, R⁴, R⁵, R⁶, R⁷, R¹¹, R¹², R¹³ et R¹⁴ est un OMEGA, chaque OMEGA est identique ou différent ;
à condition qu'au moins un des R³, R⁴, R⁵, R⁶, R⁷, R¹¹, R¹², R¹³ et R¹⁴ soit un OMEGA ; ou est
B) un colorant de cyanine asymétrique substitué de manière cyclique, comprenant un premier système hétérocyclique qui est un benzothiazolium, benzoxazolium, benzosélénazolium, benzimidazolium ou dialkylindolinium substitué ; qui est lié par une partie de pont monométhine, triméthine ou pentaméthine attachée en position 2 dudit premier système cyclique en position 2 ou 4 d'un second système hétérocyclique qui est un pyridinium ou quinolinium substitué, dans lequel un ou plusieurs substituants dudit second système cyclique est un OMEGA, où OMEGA est un substituant cyclique, saturé ou insaturé, substitué ou non substitué, qui a un total de 2-16 atomes de carbone cycliques dans les 1-2 cycles alicycliques, hétéroalicycliques, aromatiques ou hétéroaromatiques, contenant 1-4 hétéroatomes (dans lequel les hétéroatomes sont O, N ou S).

13. Complexe selon la revendication 12, dans lequel le composé de colorant est comme défini dans une des revendications 1 à 5 ou 6 à 11.

14. Procédé de coloration d'acides nucléiques dans un échantillon, comprenant :
a) la combinaison d'un échantillon qui contient ou est supposé contenir des acides nucléiques avec un composé de colorant asymétrique substitué de manière cyclique comme défini dans la revendication 12 ou la revendication 13 ou l'une quelconque des revendications 1 à 11 ;
b) l'incubation de l'échantillon pendant un temps suffisant pour que le composé de colorant se combine avec les acides nucléiques dans l'échantillon pour former un complexe acide nucléique-colorant qui donne un signal fluorescent détectable ;
c) l'observation du signal fluorescent détectable du complexe acide nucléique-colorant.

15. Procédé selon la revendication 14, dans lequel :
(i) les acides nucléiques sont enfermés dans une structure biologique ; ou
(ii) les acides nucléiques sont enfermés dans une structure biologique et le procédé comprend en outre la combinaison de l'échantillon avec un ou plusieurs colorants supplémentaires, seuls ou en combinaison ; où au moins un colorant supplémentaire est un colorant d'acide nucléique fluorescent qui pénètre ou ne pénètre pas les cellules ; un autre colorant supplémentaire pénètre sélectivement les bactéries Gram positives ou Gram négatives ; et où chacun des colorants supplémentaires a une réponse fluorescente à l'illumination qui est de manière détectable différente de celle des autres colorants.

16. Procédé selon la revendication 15(ii) dans lequel
(i) le colorant d'acide nucléique ne pénètre pas les cellules avec membranes intactes ; ou
(ii) le colorant d'acide nucléique ne pénètre pas les cellules avec des membranes intactes et est un dérivé de monomère ou de dimère de phénanthridium qui est un éthidium, un dimère d'éthidium ou propidium ; ou un dérivé de monomère ou de dimère de benzazolium.

17. Procédé selon la revendication 15(ii) dans lequel le colorant d'acide nucléique pénètre les cellules ou pénètre sélectivement les bactéries Gram positives et est un phénanthridium substitué par un alkyle en C₄-C₈, ou 2'-(4-hydroxyphényl)-5-(4-méthyl-1-pipérazinyl)-2,5'-bi-1H-benzimidazole, 2'-(4-éthoxy-phényl)-5-(4-méthyl-1-pipérazinyl)-2,5'-bi-1H-benzimidazole, ou dichlorhydrate de 4',6-diamidino-2-phénylindole.

18. Procédé selon la revendication 14, dans lequel les acides nucléiques sont en solution.

19. Procédé selon la revendication 14 ou 18 comprenant en outre la séparation électrophorétique des acides nucléiques ou du complexe acide nucléique-colorant et facultativement comprenant en outre la combinaison du composé de colorant avec le gel avant ou après l'électrophorèse.

20. Procédé selon la revendication 14, de coloration d'acides nucléiques dans un échantillon, dans lequel les acides nucléiques sont immobilisés sur un support solide ou semi-solide.

21. Procédé selon l'une quelconque des revendications 14, 15(i), 15(ii), 16(i), 16(ii) ou 17 comprenant en outre :
(i) la combinaison de l'échantillon avec un ou plusieurs colorants supplémentaires, seuls ou en combinaison ; où au moins un colorant supplémentaire est un colorant fluorescent d'un poids moléculaire de moins de 2000 qui colore sélectivement une structure cellulaire qui n'est pas un acide nucléique ou n'est pas un substrat enzymatique ; et où chacun des colorants supplémentaires a une réponse fluorescente à l'illumination qui est de manière détectable différente de celle des autres colorants, dans lequel la structure cellulaire facultativement est une membrane cellulaire, une protéine, une vacuole, une mitochondrie, un appareil de Golgi, un réticulum endoplasmique, un cytoplasme, un lysosome, ou un saccharide ou polysaccharide ;
(ii) la combinaison de l'échantillon avec un ou plusieurs colorants supplémentaires, seuls ou en combinaison ; où au moins un colorant supplémentaire est un peptide ou une protéine fluorescente, où la protéine est un anticorps, une lectine, une avidine, la streptavidine, la protéine A ou la protéine G ; et où chacun des colorants supplémentaires a une réponse fluorescente à l'illumination qui est de manière détectable différente de celle des autres colorants ; ou
(iii) la combinaison de l'échantillon avec un ou plusieurs colorants supplémentaires, seuls ou en combinaison ; où au moins un colorant supplémentaire est un substrat fluorogénique pour une enzyme intracellulaire ; où ledit substrat a une réponse fluorescente à l'illumination qui est de manière détectable différente de celle des autres colorants après action de l'enzyme, dans lequel l'enzyme intracellulaire facultativement est une enzyme hydrolytique, une oxydase ou une réductase.

22. Procédé selon l'une quelconque des revendications 15(ii), 16(i), 16(ii) ou 17 dans lequel la réponse fluorescente des colorants à l'illumination est détectée en utilisant un cytomètre de flux, un fluoromètre ou un lecteur de plaques de fluorescence, ou un microscope à fluorescence.

23. Composé de formule 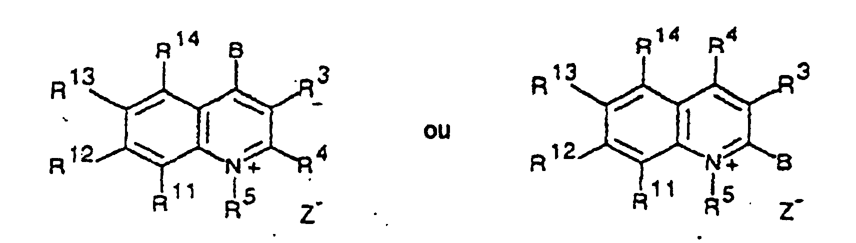 dans laquelle
R⁵ est un OMEGA comme défini dans la revendication 6 ou la revendication 9 ;
B est méthyle ;
R³, R¹¹, R¹², R¹³ et R¹⁴ sont indépendamment H ou alkyle ayant 1-6 carbones ; et
R⁴ est F, Cl, Br, I ou -OSO₂R¹⁹ où R¹⁹ est alkyle ayant 1-6 carbones, ou aryle.

24. Composé de formule 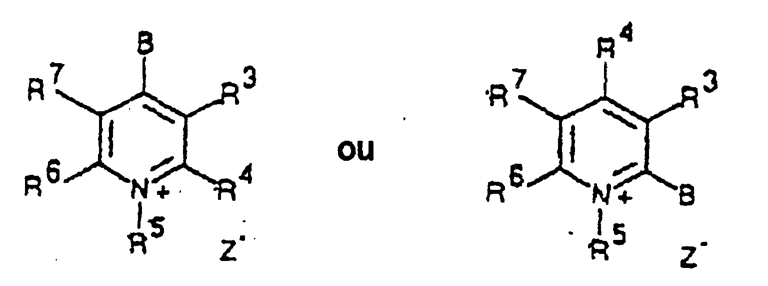 ou de formule 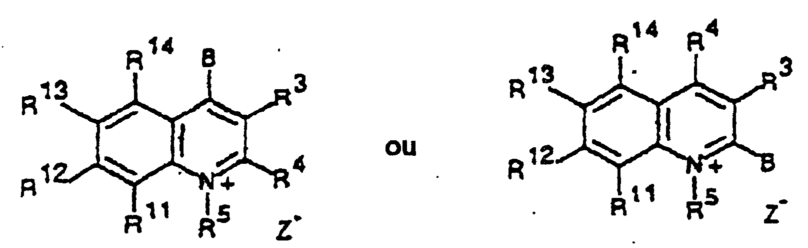 dans laquelle
R⁵ est un OMEGA comme défini dans la revendication 6 ou la revendication 9 ;
B est méthyle ;
R⁶ et R⁷ sont H ;
R³, R¹¹, R¹², R¹³ et R¹⁴ sont indépendamment H ou alkyle ayant 1-6 carbones ; et
R⁴ est F, Cl, Br, I ou -OSO₂R¹⁹ où R¹⁹ est alkyle ayant 1-6 carbones, ou perfluoroalkyle ayant 1-6 carbones ou aryle.

25. Composé selon la revendication 23 ou la revendication 24, quand substitué avec un groupement formant en position 2 une liaison éther, une liaison thioéther ou un groupement amino (soit substitué soit non substitué).

26. Procédé de préparation d'un colorant fluorescent, comprenant la liaison d'un composé de pyridinium ou de quinolinium comme défini dans la revendication 23 ou la revendication 24 avec un composé de benzazolium de formule 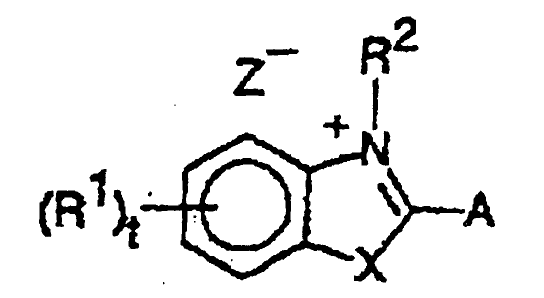 dans laquelle R¹, t, X et Z sont comme définis dans la revendication 1 et A est un substituant approprié pour la liaison des composés, pour former un composé comprenant un système cyclique de benzazolium lié en position 2 par 1, 3 ou 5 groupements méthine de liaison à la position 4 d'un système cyclique de pyridinium ou de quinolinium, et, quand le composé de pyridinium ou de quinolinium est comme défini dans la revendication 22 ou la revendication 23, le procédé facultativement en outre comprenant la condensation du système cyclique de pyridinium ou de quinolinium en position 2 avec un alcool, un phénol, un alkoxyde, un thiol, un thiophénol, un thioéther, l'ammoniaque ou une amine.

27. Procédé selon la revendication 26 dans lequel le colorant fluorescent résultant a en position 2 du système cyclique de pyridinium ou de quinolinium un groupement substituant formant une liaison éther, une liaison thioéther ou un groupement amino (soit substitué soit non substitué).

28. procédé selon la revendication 27, dans lequel le groupement substitué en position 2 du système cyclique de pyridinium ou de quinolinium comprend, en plus de n'importe quel groupement amino directement lié à la position 2 (le groupement amino de là position 2), un groupement amino substitué, où le groupement amino substitué est un cycle hétéroalicyclique qui a de 3 à 16 atomes cycliques et qui est lié à des atomes O, N ou S liés à la position 2 du système cyclique, et facultativement où il y a un groupement amino de la position 2 et le groupement amino substitué est le groupement NR¹⁰ dans une partie de la formule -(CH₂)₂-NR¹⁰-(CH₂)₂- cycle directement lié à l'atome N du groupement amino de la position 2, R¹⁰ étant H ou alkyle en C₁-C₆.

29. Utilisation, pour pénétrer des structures biologiques en détectant les acides nucléiques, d'un composé comme défini dans la revendication 12 ou la revendication 13 ou une quelconque des revendications 1-11, dans laquelle la structure biologique facultativement est :
(i) une particule virale ;
(ii) une organelle, ou
(iii) une cellule.

30. Utilisation selon la revendication 29, dans laquelle le cycle de pyridinium (groupement Q des revendications 1 à 5) est de la formule 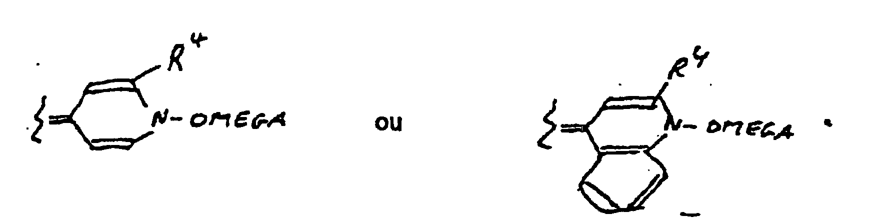 dans laquelle
OMEGA est comme défini dans la revendication 1 ou est un groupement phényle et R⁴ est
(i) comme défini dans la revendication 1, ou
(ii) choisi parmi H, F, Cl, Br, n-butyle, -NH-phényle, NEt₂, N-nPr₂, N-méthylpipérazyle, morpholinyle, anilinyle, 2-(N-3-diméthyl-aminopropyl)-N-propylamino, Me, OMe, OEt, OPh, Ph, SMe, O-iPr, cyclohexyle, S-2-pyridyle, 2-(2-diméthylaminoéthylthio) et OSO₂CF₃.
